(19) Europäisches Patentamt
European Patent Office
Office européen des brevets

(11) Veröffentlichungsnummer: **0 056 962**
**B1**

# (12) EUROPÄISCHE PATENTSCHRIFT

(45) Veröffentlichungstag der Patentschrift: **02.10.85**

(51) Int. Cl.⁴: **C 07 D 405/06**, A 61 K 31/41

(21) Anmeldenummer: **82100279.7**

(22) Anmeldetag: **16.01.82**

## (54) Neue N-substituierte Polyglycidyl-urazolverbindungen, Verfahren zu ihrer Herstellung sowie pharmazeutische Zubereitungen.

(30) Priorität: **24.01.81 DE 3102373**

(43) Veröffentlichungstag der Anmeldung: **04.08.82 Patentblatt 82/31**

(45) Bekanntmachung des Hinweises auf die Patenterteilung: **02.10.85 Patentblatt 85/40**

(84) Benannte Vertragsstaaten: **AT BE CH DE FR GB IT LI LU NL SE**

(56) Entgegenhaltungen:
**EP - A - 0 026 312**
**EP - A - 0 033 503**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Fischer, Herbert, Dr., Am Nettchesfeld 14, D-4000 Düsseldorf 13 (DE)**
Erfinder: **Hase, geb. Kornrumpf, Brigitte, Dr., Millrather Weg 29, D-4006 Erkrath 1 (DE)**
Erfinder: **Möller, Hinrich, Dr., Schumannstrasse 11, D-4019 Monheim (DE)**
Erfinder: **Wilk, Hans-Christoph, Dr., An der Obererft 94, D-4040 Neuss (DE)**
Erfinder: **Zeidler, Ulrich, Dr., Heinrich-Lersch-Strasse 19, D-4000 Düsseldorf 13 (DE)**

**EP 0 056 962 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der DE-OS 2 907 349 sind Arzneimittelzubereitungen mit cytostatischer Wirksamkeit, die als pharmakologischen Wirkstoff Triglycidylisocyanurat (TGI) und/oder solche TGI-Derivate enthalten, in denen das Wasserstoffatom des Kohlenstoffs in 2-Stellung der Glycidylgruppe durch einen Alkylrest mit 1 bis 4 Kohlenstoffatomen ersetzt sein kann. Verbindungen dieser Art zeichnen sich dadurch aus, daß die drei N-Atome des Isocyanursäurerings mit Epoxygruppen enthaltenden Glycidylresten substituiert sind, die in 2-Stellung auch mit einem Alkylrest mit 1 bis 4 Kohlenstoffatomen substituiert sein können.

Gegenstand der DE-OS 3 037 094.6 sind unter anderem Arzneimittelzubereitungen mit cytostatischer Wirksamkeit, enthaltend Verbindungen der allgemeinen Formel

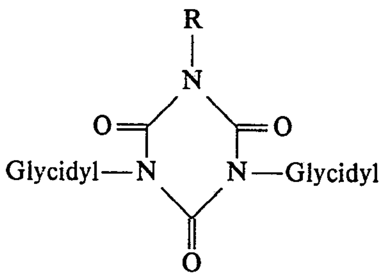

in der R die folgende Bedeutung haben kann: Alkyl, Aryl, Aralkyl, Alkaryl, Cycloalkyl, welche Reste gewünschtenfalls auch heterocyclischer Natur, ungesättigt und/oder mit wenigstens einem der folgenden Substituenten substituiert sein können: Halogen, Hydroxyl, Amino, N-substituiertes Amino, Mercapto, Alkylmercapto, Arylmercapto, Alkylsulfoxy, Arylsulfoxy, Alkoxy, Aryloxy, Acyloxy und heterocyclischer Rest. Der Glycidylrest hat die zuvor angegebene Bedeutung.

In der EP-A-33 503 sind ebenfalls mehrfach mit Glycidylgruppen substituierte N-heterocyclische Verbindungen mit cytostatischer Wirkung beschrieben.

Die vorliegende Erfindung geht von der Feststellung aus, daß strukturanaloge Verbindungen, die sich jedoch vom Urazol als Grundkörper ableiten, ebenfalls eine überraschend starke cytostatische Wirksamkeit entfalten, die die Wirksamkeit von TGI sogar übertreffen kann.

Gegenstand der Erfindung sind dementsprechend in einer ersten Ausführungsform neue N-substituierte Polyglycidyl-urazolverbindungen der allgemeinen Formel I

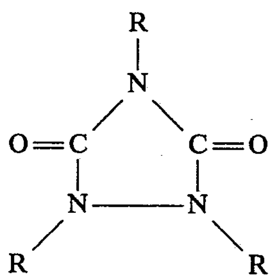

(I)

in der die Reste R einen Glycidylrest der allgemeinen Formel II

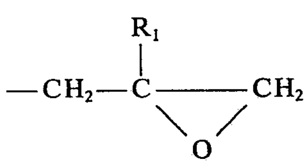

(II)

bedeuten, worin $R_1$ Wasserstoff oder einen Alkylrest mit 1 bis 4 C-Atomen ist, oder zwei der Reste R einen Glycidylrest der allgemeinen Formel II bedeuten und dann der verbleibende Rest R ein Rest Z ist, der 1 bis 15 C-Atome, Wasserstoff und gegebenenfalls bis zu 3 Heteroatome enthält zur Anwendung als cytostatische Mittel.

Ein weiterer Gegenstand der Erfindung sind die N-substituierten-Polyglycidyl-urazolverbindungen ansich. Dabei sind jedoch für die Vertragsstaaten CH, DE, FR, GB, IT und LI Verbindungen ausgenommen, bei denen alle drei in N-Substituation vorliegenden Reste R Glycidylreste der allgemeinen

Formel II mit $R_1$ = Wasserstoff oder Methylgruppen bedeuten. Diese Verbindungen sind Gegenstand der nicht vorveröffentlichten europäischen Patentanmeldung 0 026 312. Sie sind für die Vertragsstaaten AT, BE, LU, NL und SE eine bevorzugte Ausführungsform der Erfindung. In jedem Falle ist jedoch ihre Anwendung als cytostatische Mittel eine besonders bevorzugte Ausführungsform der Erfindung.

In einer weiteren wichtigen Ausführungsform der Erfindung liegen am Urazolring nur zwei Glycidylreste der allgemeinen Formel II vor, während der dritte Rest ein Rest Z mit der angegebenen Bedeutung ist. Auch in diesem Fall ist es weiterhin bevorzugt, daß die beiden Glycidylreste in $R_1$ Wasserstoff enthalten. Z kann grundsätzlich ein beliebiger organischer Rest sein, der neben bis zu 12 Kohlenstoff- und Wasserstoffatomen auch bis zu 3 Heteroatome enthalten kann. Als Heteroatome kommen in erster Linie O, N, S und/oder P in Betracht.

In bevorzugten Ausführungsformen der Erfindung kann Z eine der folgenden Bedeutungen haben: Alkyl, Aryl, Aralkyl, Cycloalkyl, welche Reste gewünschtenfalls auch heterocylischer Natur, ungesättigt und/oder mit wenigstens einem Substituenten substituiert sein können. Als Substituenten kommen insbesondere in Betracht: Halogen, Hydroxyl, Amino, N-substituiertes Amino, Mercapto, Alkylmercapto, Arylmercapto, Alkylsulfoxy, Arylsulfoxy, Alkoxy, Aryloxy, Acyloxy und heterocyclischer Rest. Liegt in Z ein substituierter Rest vor, so können entsprechend substituierte Alkylreste bevorzugt sein, substituierte Arylreste beziehungsweise Cycloalkylreste sind jedoch nicht ausgeschlossen. Als ungesättigte Reste sind olefinisch ungesättigte Reste bevorzugt.

Gegenstand der Erfindung sind weiterhin Verfahren zur Herstellung der neuen N-substituierten Polyglycidylverbindungen der allgemeinen Formel I sowie Arzneimittelzubereitungen mit cytostatischer Wirksamkeit, enthaltend Verbindungen der allgemeinen Formel I.

Der Wirkungsmechanismus der im Rahmen der Erfindung eingesetzten Verbindungen ist im einzelnen nicht geklärt. Vermutlich sind die auch hier wie beim Triglycidylisocyanurat der DE-OS 2 907 349 vorliegenden Glycidylgruppen für die cytostatische Wirkung von herausragender Bedeutung. Alle erfindungsgemäß beschriebenen Verbindungen der allgemeinen Formel I kennzeichnen sich durch das Vorliegen von wenigstens 2 solcher Gycidylgruppen. Daneben liegt gegebenenfalls der in weitem Rahmen varierbare Rest Z in der betroffenen Verbindungsklasse vor. Es ist möglich, daß über diesen Rest Z Einfluß auf die Verteilung von lipophilen und hydrophilen Präferenzen genommen wird und daß damit in gewissem Ausmaß die Aufnahme der Verbindungen durch den Organismus gesteuert werden kann. Die Bedeutung des erfindungsgemäß eingeführten neuen Substituenten Z muß sich aber nicht darauf beschränken.

Der Rest Z ist gemäß seiner vorher gegebenen Definition ein Kohlenwasserstoffrest, der auch bis zu 3 Heteroatome enthalten kann. Als Heteroatome kommen insbesondere N, O, S und/oder P in Betracht. Dieser Rest enthält insgesamt nicht mehr als 15 Kohlenstoffatome, vorzugsweise nicht mehr als 8 Kohlenstoffatome. Interessant können insbesondere Reste sein, die bis zu 6 oder vorzugsweise sogar nur bis zu 4 Kohlenstoffatome enthalten, wobei diese Zahlenwerte unabhängig von der jeweiligen Struktur zu verstehen sind und sich lediglich auf die Summe aller Kohlenstoffatome im betroffenen Rest beziehen.

Bedeutet Z einen Aryl-, Aralkyl- oder Alkarylrest, so sind hier insbesondere 1-kernige Substituenten bevorzugt. Typische Vertreter sind Phenyl, Benzyl, Tolyl, Xylyl und verwandte Verbindungen. Auch bei den cycloaliphatischen Ringen für den Rest Z sind einkernige Ringsysteme auf der Basis von Cyclopentyl, Cyclohexyl und ihren Abkömmlingen bevorzugt. Entsprechende heterocyclische Reste, insbesondere also 1-kernige Ringverbindungen mit O, N und/oder S im System fallen in den Rahmen der Erfindung. Die Ringsysteme können dabei 1, 2 oder 3 solcher Heteroatome enthalten. Diese heterodyclischen Reste enthalten vorzugsweise 5 oder 6 Ringglieder. Gewünschtenfalls können alle hier genannten ringförmigen Substituenten, seien sie aromatischer oder cycloaliphatischer Natur, ihrerseits weitere Substituenten aufweisen. Geeignete Substituenten sind beispielsweise Halogen, Hydroxyl oder Alkoxy.

In einer besonders bevorzugten Ausführungsform der Erfindung bedeutet der Rest Z einen gegebenenfalls substituierten Alkylrest. Dieser Alkylrest kann gradkettig oder verzweigt und gesättigt oder ungesättigt sein und enthält — unter Ausschluß seiner Substituenten nicht mehr als 10, insbesondere nicht mehr als 8, Kohlenstoffatome. In dieser Ausführungsform der Erfindung sind insbesondere solche Verbindungen der allgemeinen Formel I bevorzugt, in denen der Rest Z unsubstituiertes Alkyl mit 1 bis 6 C-Atomen oder einen entsprechenden Alkylrest bedeutet, der mit Halogen, Hydroxyl, Amino, N-substituiertem Amino, Mercapto, Alkylmercapto, Arylmercapto, Alkylsulfoxy, Arylsulfoxy, Alkoxy, Aryloxy und/oder Acyloxy substituiert ist, wobei der Substituent auch heterocyclischer Natur sein kann.

Hierbei können derart substituierte Reste ein- oder mehrfach mit den genannten Gruppen substituiert sein. Bevorzugt liegen 1 bis 3 der genannten Substituenten am jeweiligen Rest Z vor, wobei in einem besonders bevorzugten Fall solche substituierte Alkylreste der genannten Art enthaltende Verbindungen der allgemeinen Formel I in den erfindungsgemäßen Arzneimittelzubereitungen eingesetzt werden.

Liegen an dem substituierten Alkylrest Z substituierende Gruppen vor, die ihrerseits Kohlenwasserstoffreste enthalten — insbesondere also im Falle der Reste N-substituiertes Amino, Alkylmercapto, Arylmercapto, Alkylsulfoxy, Arylsulfoxy, Alkoxy, Aryloxy und Acyloxy — so weisen diese substituierenden Gruppen vorzugsweise nicht mehr als 10, zweckmäßigerweise nicht mehr als 8, C-Atome auf. Die besonders bevorzugte Grenze liegt hier bei 6 C-Atomen, insbesondere bei nicht mehr als 4 C-Atomen. Diese substituierenden Kohlenwasserstoffreste können ihrerseits Aryl, Aralkyl, Alkaryl, Cycloalkyl und/oder Alkylreste sein, die gewünschtenfalls auch Substituenten wie Halogen, Hydroxyl oder Alkoxy aufweisen können. Auch hier sind Heteroatome enthaltende Reste der zuvor geschilderten Art, also beispielsweise heterocyclische Ringsysteme mit 1 bis 3 Heteroatomen der zuvor geschilderten Art — insbesondere 1-kernige Ringe mit N, O und/oder S als Heteroatomen — eingeschlossen. Entsprechende 5- beziehungsweise 6-gliedrige Heterocyclen sind bevorzugt.

Eine besonders bevorzugte Ausführungsform der Erfindung betrifft Verbindungen der allgemeinen Formel I, in denen einer der Reste R — das heißt der Rest Z — einen mono- oder disubstituierten Alkylrest der genannten Art bedeutet, der aus der folgenden Gruppe ausgewählt ist: Monohydroxyalkyl, Dihydroxyalkyl, Halogenhydroxyalkyl, N-substituiertes Aminohydroxyalkyl, Alkylmercaptohydroxyalkyl, substituiertes Alkylmercaptohydroxyalkyl, die entsprechenden Alkylsulfoxyhydroxyalkyle, gegebenenfalls substituiertes Alkoxyhydroxyalkyl und gegebenenfalls substituiertes Acyloxyhydroxyalkyl. Bevorzugt kann dabei der Alkylrest bis zu 7, vorzugsweise 3 bis 7 und insbesondere 3, 4 oder 5 Kohlenstoffatome enthalten.

Im Rahmen der Erfindung können Verbindungen der allgemeinen Formel I bevorzugt sein, in denen der Rest Z gradkettiges oder verzweigtes unsubstituiertes Alkyl mit bis zu 6, vorzugsweise mit bis zu 4, Kohlenstoffatomen bedeutet. In Betracht kommen insbesondere die Reste Methyl, Ethyl, Propyl, Isopropyl sowie die entsprechenden $C_4$-Reste und ihre olefinisch ungesättigten Analogen. Weiterhin sind ganz besonders solche Verbindungen der allgemeinen Formel I bevorzugt, in denen einer der Reste R ein mono- oder disubstituierter Alkylrest der genannten Art mit insbesondere 3 Kohlenstoffatomen ist und dabei vorzugsweise wenigstens eine Hydroxylgruppe aufweist. Hier liegt also dann stets wenigstens eine Hydroxygruppe — vorzugsweise neben einem weiteren Substituenten an diesem Rest — vor, während die beiden anderen Reste R den Glycidylrest der allgemeinen Formel II bedeuten.

Diese substituierenden Gruppen im Rest Z verteilen sich dabei in einer weiterhin bevorzugten Ausführungsform der Erfindung auf die 2- und die 3-Stellung des betroffenen Restes R. Dabei kann die Hydroxygruppe entweder in der 2-Stellung oder in der 3-Stellung vorliegen. Weiterhin besonders bevorzugt sind entsprechend substituierte Verbindungen der Formel I, die im Rest Z neben der Hydroxylgruppe keine weitere substituierende Gruppe oder als weiteren Substituenten Hydroxyl, Halogen, einen N-substituierten Aminorest, einen gegebenenfalls substituierten Alkoxyrest, einen gegebenenfalls substituierten Alkylmercapto- beziehungsweise Alkylsulfoxyrest oder einen gegebenenfalls substituierten Acyloxyrest aufweisen. Als Halogen sind insbesondere Chlor und/oder Brom bevorzugt, Fluor und Jod sind jedoch nicht ausgeschlossen. Die N-substituierten Aminoreste können den Formeln

$$-NHR_2 \text{ oder } -NR_2R_3 \qquad (III)$$

entsprechen. Hierbei sind die Reste $R_2$ beziehungsweise $R_3$ Kohlenwasserstoffreste, die ihrerseits substituiert sein können. In der bevorzugten Ausführungsform der Erfindung enthalten diese Reste $R_2$ und gewünschtenfalls $R_3$ bis zu 12 Kohlenstoffatome, wobei bei der Disubstitution am Stickstoff die Summe der Reste $R_2$ und $R_3$ vorzugsweise 12 Kohlenstoffatome nicht überschreitet. Besonders bevorzugt enthalten diese Substituenten $R_2$ und $R_3$ insgesamt bis zu 8 und dabei insbesondere nicht mehr als 5 Kohlenstoffatome. Die Reste $R_2$ und $R_3$ können auch zu einem gesättigten oder ungesättigten, gegebenenfalls aromatischen und/oder heterocyclischen Ring zusammengeschlossen sein. Bevorzugt können $R_2$ und gegebenenfalls $R_3$ auch Alkylreste sein. Sind diese Reste ihrerseits nochmals substituiert, so sind im Rahmen der Erfindung als solche Substituenten insbesondere Hydroxyl, Alkoxy beziehungsweise Halogen — vorzugsweise Chlor oder Brom — vorgesehen. Liegt neben der Hydroxylgruppe in Z ein Acyloxyrest, ein Alkoxyrest oder ein Alkylmercapto- beziehungsweise Alkylsulfoxyrest vor, so enthält vorzugsweise dieser Rest ebenfalls bis maximal 10 C-Atome, wobei auch hier die bevorzugte Grenze bei 8 C-Atomen liegt und besonders bevorzugt ist, nicht mehr als 5 C-Atome an dieser Stelle in das Molekül einzuführen. Bevorzugt sind auch bei Acyloxyresten Alkylreste mit der entsprechenden Kohlenstoffzahl, wenn auch Arylreste nicht ausgeschlossen sind. Die Acyloxyreste leiten sich bevorzugt von Monocarbonsäuren der genannten Kohlenstoffzahl und Struktur ab.

Insgesamt weist der Rest Z jedoch höchstens 15 C-Atome auf.

Die erfindungsgemäßen Arzneimittel können bevorzugt einzelne definierte Verbindungen der allgemeinen Formel I enthalten, es hat sich aber gezeigt, daß auch Wirkstoffgemische mehrerer unter die allgemeine Formel I fallender Verbindungen hochwirksame Cytostatica sind. Im Rahmen der Erfindung kann es weiterhin bevorzugt sein, einzelne bestimmte oder eine Mischung mehrerer Verbindungen der erfindungsgemäßen Definition gemäß Formel I in Abmischung mit den TGI-Verbindungen gemäß der erwähnten deutschen Patentanmeldung DE-OS 2 907 349 beziehungsweise der deutschen Patentanmeldung P 3 037 094.6 zu verwenden. Die Herstellung der erfindungsgemäßen Verbindungen kann in mehrfacher Weise erfolgen und bildet einen weiteren Gegenstand der Erfindung:

1. Einführung der Glycidylgruppen der allgemeinen Formel II an den Urazolring in die N-Substitution. Hierzu wird zunächst in an sich bekannter Weise Urazol hergestellt, das heißt die der Formel I entsprechende Grundverbindung, in der jedoch anstelle der N-Glycidylgruppierungen jeweils eine —NH-Gruppierung vorliegt. Es erfolgt dann der Ersatz dieses Wasserstoffs am Stickstoff durch die Glycidylgruppe.

Für diese abschließende Reaktionsstufe bestehen vor allem zwei grundsätzliche Möglichkeiten. Die eine liegt in der direkten Einführung der Glycidylgruppierung durch Umsetzung der NH-Gruppierung mit Epi-Halohydrinen, insbesondere Epi-Chlorhydrin oder Epi-Bromhydrin und anschließende Dehydrohalogenierung. Der andere Weg vervollständigt den Molekülaufbau in den zwei Reaktionsschritten: Es werden zunächst die entsprechenden allylsubstituierten Vorprodukte gebildet, woraufhin in einer abschließenden Verfahrensstufe die Allylgruppe epoxidiert wird.

Zur Umsetzung von —NH-Gruppen mit Epi-Halogydrinen besteht eine umfangreiche Literatur. Die Umsetzung kann in Gegenwart einer geringen Menge einer quartären Ammoniumverbindung als Katalysator durchgeführt werden (vergleiche hierzu beispielsweise Houben-Weyl »Methoden der organischen Chemie« Band 14/2 (1963), 497, 547). Besonders geeignete quartäre Ammoniumverbindungen zählen zur Klasse der Phasentransfer-Katalysatoren. Hierbei handelt es sich bekanntlich um quartäre Ammoniumverbindungen mit erhöhter Lipophilie, die insbesondere durch die Gegenwart hinreichend großer organischer Reste in der quartären Ammoniumverbindung sichergestellt ist. Ausführliche Angaben zu den Phasentransfer-Katalysatoren finden sich beispielsweise in W.P. Weber, G.W. Gobel »Phase Transfer Catalysis in Organic Synthesis«, Springer-Verlag, Berlin, Heidelberg, New York, 1977 sowie E.V. Dehmlow, S.S. Dehmlow »Phase Transfer Catalysis«, Verlag Chemie, Weinheim, Deerfield Beech (Florida), Basel, 1980.

Die Phasentransfer-Katalysatoren werden vorzugsweise in Mengen von etwa 0,1 bis 10 Gewichtsprozent, insbesondere 0,5 bis 5 Gewichtsprozent, und insbesondere 0,5 bis 3 Gewichtsprozent, bezogen auf die vorgelegte Urazolverbindung mitverwendet. In der nachfolgenden Reaktionsstufe wird die Dehydrohalogenierung, die teilweise auch durch überschüssiges Epihalohydrin abläuft, durch Zusatz von Basen, vorzugsweise Alkalihydroxide, vervollständigt.

Bei dem zweiten Verfahren wird die Urazolverbindung nicht unmittelbar mit der Epoxidverbindung umgesetzt. Statt dessen erfolgt zunächst ihre Umsetzung mit Allylhalogeniden, die dem Glycidylrest der Verbindungen der allgemeinen Formel I entsprechen, jedoch anstelle der Epoxidgruppe eine olefinische Doppelbindung aufweisen, woraufhin die entstandenen allylsubstituierten Urazole epoxidiert werden. Die Epoxidation kann in an sich bekannter Weise mit Persäuren vorgenommen werden. Als verwandte Umsetzung ist beispielsweise die Umsetzung von Cyanursäure mit Allylhalogeniden beschrieben in der US-PS 3 376 301, die Epoxidation von Allylisocyanuraten mit Persäuren ist zum Beispiel in Houben-Weyl aaO, Band 6/3, 385 ff beschrieben. Sie kann zum Beispiel in Gegenwart einer geringen Menge einer quartären Ammoniumverbindung als Katalysator durchgeführt werden.

Die Umsetzung des Urazols beziehungsweise der monosubstituierten Urazolverbindung (vergleiche hierzu im folgenden unter Ziffer 3.) mit Epi-Halohydrinen beziehungsweise Allylhalogeniden erfolgt zweckmäßigerweise im Temperaturbereich von etwa 50 bis 150° C, vorzugsweise von etwa 70 bis etwa 125° C.

Die Reaktion kann in einem Überschuß der Epi-Halohydrinverbindung als Lösungsmittel oder in polaren aprotischen Lösungsmitteln vorgenommen werden, die wenigstens einen der Reaktionspartner teilweise lösen und den Reaktanten gegenüber nicht reaktiv sind. Ein besonders zweckmäßiges Lösungsmittel ist die Klasse der Dialkylformamide, insbesondere der niederen Dialkylformamide wie Dimethylformamid. Die bevorzugte Reaktionszeit beträgt 1 bis 10 Stunden, insbesondere 2 bis 5 Stunden.

Die vollständige Dehydrohalogenierung der intermediär entstandenen Halohydrine kann durch Zugabe von festem, gepulvertem Alkali, vorzugsweise NaOH, oder von hochkonzentrierten wäßrigen Lösungen vorgenommen werden. Diese Halogenwasserstoffabspaltung wird entweder in überschüssigem Epi-Halohydrin oder nach Abdestillieren desselben unter vermindertem Druck in einem polaren aprotischen Lösungsmittel wie zum Beispiel Dimethoxyethan, Diglyme oder Dimethylformamid bei Temperaturen zwischen —10° und 60° C, vorzugsweise zwischen 0 und 45° C durchgeführt.

Auch die Epoxidation der Allylgruppierungen mittels Persäuren wird vorzugsweise in Lösungsmitteln durchgeführt. Geeignet sind auch hier polare Lösungsmittel, beispielsweise Halogenkohlenwasserstoffe oder Alkohole. Die geeignete Reaktionstemperatur liegt üblicherweise im Bereich von 0 bis 50° C, insbesondere zwischen etwa 10 und 30° C. Die Persäure wird zweckmäßigerweise in annähernd äquivalenter Menge oder nur in leichtem Überschuß eingesetzt. m-Chlorperbenzoesäure ist als Handelsprodukt leicht zugänglich und für die Durchführung der Reaktion geeignet. Die Reaktionsdauer liegt in der Regel im Bereich von 24 Stunden oder mehr, beispielsweise bis zu 48 Stunden.

Wird bei diesen Reaktionen von nicht substituiertem Urazol als Ausgangsmaterial ausgegangen, so können die Tri-glycidyl-substituierten Urazole erhalten werden, deren Herstellung wird für die Vertragsstaaten CH, DE, FR, GB, IT und LI nicht beansprucht.

2. Zur Herstellung von erfindungsgemäßen Urazolderivaten, in denen zwei Reste R aus der allgemeinen Formel I einen Glycidylrest und der dritte Rest R den Rest Z bedeuten, sind verschiedene Herstellungswege möglich. Eine Möglichkeit ist die Umsetzung von Triglycidylurazol (TGU) mit einem Unterschuß an Wasser, Alkohol, primären und/oder sekundären Aminen, Mercaptanen, Iminen, Imiden,

Carbonsäuren, Halogenwasserstoff oder Wasserstoff.

Auf Grund der Ähnlichkeit der drei Glycidylgruppen im TGU führt diese Reaktion zunächst immer zu Produktgemischen, die ihrerseits therapeutisch wirksam sein können. Es ist aber auch möglich und Teil des im folgenden geschilderten Verfahrens aus diesen Gemischen die entsprechenden Verbindungen der allgemeinen Formel I durch geeignete Trennverfahren, zum Beispiel präparative Dünnschichtchromatographie oder Säulenchromatographie abzutrennen.

Im Rahmen dieser Umsetzungen wird eine Glycidylgruppe zum Rest Z der Verbindungen der allgemeinen Formel I umgewandelt. Bei der reduktiven Behandlung der Glycidylgruppe mit Wasserstoff beziehungsweise Wasserstoff liefernden Verbindungen entsteht ein Monohydroxyalkylrest Z. Als Wasserstoff liefernde Verbindungen können beispielsweise Hydridverbindungen, beispielsweise komplexe Borhydride wie Natriumborhydrid Verwendung finden. In den anderen genannten Fällen wird die Triglycidylausgangsverbindung mit einer nucleophilen Verbindung $H^+A^-$ im Unterschuß umgesetzt, wodurch ein disubstituierter Rest Z entsteht, der neben einer Hydroxylgruppe den Rest $A^-$ als zweiten Substituenten normalerweise am Nachbaratom zum hydroxylierten C-Atom des Restes R enthält.

Die Umsetzung der Glycidylgruppen einer strukturmäßig ähnlichen Verbindung, nämlich des Triglycidylisocyanurates (TGI) mit solchen nucleophilen Reaktionspartnern ist bekannter Stand der Technik und zum Beispiel beschrieben in Angew. Chemie 80, 851 (1968). Diese Reaktion wird jedoch im Stand der Technik gezielt an mehr als nur einer Epoxidgruppe des TGI durchgeführt und dient zum Beispiel im industriellen Rahmen der Vernetzung von Epoxidharzsystemen. Im erfindungsgemäßen Verfahren werden demgegenüber bevorzugt Verfahrensbedingungen ausgewählt, die eine möglichst weitgehende Ausbeutesteigerung in Richtung auf 1 : 1 Reaktionsprodukte zulassen sowie die anschließende Isolierung und Gewinnung dieser 1 : 1 Reaktionsprodukte unter Abtrennung nicht umgesetzter Anteile des Ausgangsmaterials und weiterführender Umsetzungsprodukte ermöglichen, die durch Reaktion von mehr als nur einer Epoxidgruppe mit dem nucleophilen Reaktionspartner entstanden sind.

Bei der Umsetzung von TGU-Verbindungen mit nucleophilen Reaktionspartnern $H^+A^-$ der zuvor geschilderten Art kann es schwierig sein, die gewünschten 1 : 1 Reaktionsprodukte in bevorzugten Ausbeuten zu erhalten, da die 3 Epoxidgruppen des Moleküls der Ausgangsverbindung in der Reaktivität etwa gleichwertig sind und sich somit häufig die gewünschte Diglycidylverbindung nicht als bevorzugtes Reaktionsprodukt bildet. Auch der Versuch durch Umsetzung des Triglycidylurazols mit einem Unterschuß an nucleophilem Reaktionspartner die gewünschte Verbindung anzureichern, erweist sich gelegentlich als schwierig.

Es wurde gefunden, daß die Herstellung der 1 : 1 Reaktionsprodukte überraschend einfach dann möglich wird, wenn man das Triglycidylurazol mit einem Überschuß und zwar vorzugsweise einem großen Überschuß des nucleophilen Reaktanten $H^+A^-$ umsetzt, die Reaktion dabei aber vorzeitig abbricht und den Überschuß des nucleophilen Reaktionspartners, nicht umgesetztes TGU und mitgebildete Di- und Triadditionsprodukte abtrennt. Das verbleibende rohe Diglycidylprodukt kann dann in konventioneller Weise, beispielsweise durch Säulenchromatographie, gereinigt werden. Vorzugsweise wird bei diesem Verfahren der nucleophile Reaktionspartner im 3- bis 30fachen Überschuß, insbesondere im 5- bis 20fachen Überschuß über die benötigte Menge eingesetzt. Die Umsetzung kann in Lösungsmitteln durchgeführt werden, gewünschtenfalls kann aber auch der Überschuß des nucleophilen Reaktionspartners als Lösungsmittel dienen. Wird mit Lösungsmitteln gearbeitet, so sollen diese zweckmäßigerweise weitgehend polar, aber unter den gewählten Verfahrensbedingungen vorzugsweise nicht reaktiv sein. Gegebenenfalls ist das Lösungsmittel auch nicht wassermischbar. Besonders geeignete Lösungsmittel sind beispielsweise halogenierte Kohlenwasserstoffe, insbesondere Chlorkohlenwasserstoffe. Die Reaktionstemperatur liegt üblicherweise zwischen etwa 30 und 120°C, vorzugsweise zwischen 40 und 100°C und wird dabei in einer besonders zweckmäßigen Ausführungsform so gewählt, daß innerhalb von 4 bis 5 Stunden der Epoxidgehalt des Reaktionsgemisches um die Hälfte abgenommen hat.

Die Reindarstellung und die Gewinnung des 2 Epoxidgruppen aufweisenden 1 : 1 Reaktionsproduktes aus dem Gemisch der Reaktanten ist hier und bei den anderen im folgenden geschilderten Verfahren in der Regel ein essentieller Schritt des erfindungsgemäßen Verfahrens. Zur Herstellung der Sulfoxyverbindungen aus den entsprechenden Mercaptoverbindungen vergleiche Houben-Weyl aaO, Band 9 (1955), 207—217 sowie Makromol. Chem. 169, 323 (1979).

3. Ein sehr elegantes allgemeines Verfahren zur Herstellung der Verbindungen der allgemeinen Formel I beruht auf der Umsetzung der mono-N-substituierten Urazolverbindung mit Epi-Halohydrinen. Die Herstellung von mono-N-substituiertem Urazol kann nach literaturbekannten Verfahren erfolgen. Zur einschlägigen Literatur wird beispielsweise verwiesen auf Org. Synthesis Vol. 51, 121 (1971).

Der in das Urazol eingeführte Substituent entspricht dabei in der Regel dem Rest Z aus den Verbindungen der allgemeinen Formel I. In nachfolgender Reaktion werden dann die beiden Glycidylgruppen eingeführt. Hierzu setzt man das monosubstituierte Urazol mit der entsprechenden Epi-Halohydrinverbindung, beispielsweise mit Epi-Chlorhydrin bei anschließender Dehydrohalogenierung oder mit Allylhalogenid bei anschließender Epoxidation der Doppelbindung um, wie es unter Ziffer 1. für das Tri-glycidylurazol beschrieben worden ist.

Gegenstand der vorliegenden Erfindung ist demgemäß in einer weiteren Ausführungsform das

Verfahren zur Herstellung von N-substituierten Polyglycidylurazolverbindungen der allgemeinen Formel I

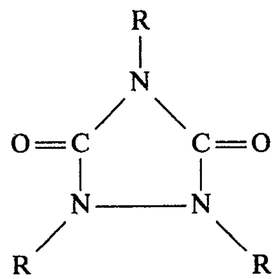

(I)

in der R die angegebene Bedeutung hat.

Dieses Verfahren ist dadurch gekennzeichnet, daß man in Urazol oder in ein mit dem Rest Z mono-N-substituiertes Urazol die beiden Glycidylreste der allgemeinen Formel II in N-Substitution einführt oder ein Triglycidylurazol mit Glycidylresten der allgemeinen Formel II einer partiellen Umsetzung mit Wasser, Alkoholen, Verbindungen mit einer primären oder sekundären Aminogruppe, Mercaptanen, Schwefelwasserstoff, Carbonsäuren, Halogenwasserstoff oder Wasserstoff beziehungsweise Wasserstoff liefernden Verbindungen unterwirft, gewünschtenfalls gebildete Thioverbindungen zu entsprechenden Sulfoxyverbindungen umwandelt und die gebildeten Reaktionsprodukte der allgemeinen Formel I aus dem Reaktionsgemisch abtrennt und als solche gewinnt.

Werden in diesem Verfahren die Glycidylreste der allgemeinen Formel II in Urazol oder in mono-N-substituiertes Urazol eingeführt, so kann dieses dadurch erfolgen, daß unmittelbar die gegebenenfalls mit dem Rest Z substituierte Urazolverbindung mit Epi-Halohydrinen umgesetzt wird, wobei diese Epi-Halohydrinverbindungen den Glycidylresten der allgemeinen Formel II entsprechen und anschließend Halogenwasserstoff abgespalten wird, oder aber es werden zunächst die Urazolverbindungen mit entsprechenden Allylhalogeniden zur Reaktion gebracht, woraufhin durch Epoxidation mit vorzugsweise Persäuren die Allylreste beziehungsweise mit $R_1$ substituierten Allylreste zur Glycidylgruppe umgewandelt werden. Zur Beschaffenheit der Verbindungen der allgemeinen Formel I, der Reste R und Z und Glycidyl und der Reaktanten zur Ausbildung dieser Reste gelten alle zuvor gemachten Angaben sinngemäß.

Liegt in den Polyglycidylverbindungen neben zwei Glycidylresten der allgemeinen Formel II ein Rest Z vor, so kann dieser in 1-, 2- oder 4-Stellung vorgesehen sein. Der 4-Stellung kann dabei insbesondere aus präparativen Gründen für diese Verbindungen mit dem Rest Z besondere Bedeutung zukommen.

Gegenstand der Erfindung ist schließlich weiterhin die Verwendung der Verbindungen der allgemeinen Formel I zur Therapie maligner Neoplasien. Einzelgaben der Verbindungen in Höhe von 1 bis 200 mg/kg können zweckmäßig sein. Es können einzelne, bestimmte Verbindungen der allgemeinen Formel I oder Mischungen von ihnen zum Einsatz kommen. Auch ihre Verwendung in Abmischung mit anderen Wirkstoffkomponenten, zum Beispiel TGI', fällt in den Rahmen der Erfindung.

Die erfindungsgemäß eingesetzten Verbindungen der allgemeinen Formel I treten in verschiedenen stereoisomeren Formen auf. Grundsätzlich eignen sich alle diese verschiedenen Formen für die Zwekke der Erfindung. Sie können dabei in Mischung oder auch in Form bestimmter isolierter Isomeren eingesetzt werden.

Zur Verwendung als Cancerostatica sollten die Wirksubstanzen mittels geeigneter Vehikel appliziert werden. Hier eignen sich die üblichen Hilfs- beziehungsweise Trägerstoffe für pharmakologische Zubereitungen. In vorliegendem Fall hat sich die Verwendung von wäßrigen Systemen, gegebenenfalls zusammen mit verträglichen Glykolethern wie Glykolmonoethylether oder Butylenglykolmethylether oder Propylenglykolmethylether bewährt, insbesondere wenn der Wirkstoff parenteral appliziert werden soll. Bei oraler Verabreichung sind die pharmazeutisch üblichen Hilfs- beziehungsweise Trägerstoff anwendbar, sofern sie eine entsprechende Verträglichkeit mit den Glycidylverbindungen aufweisen.

Im Tierexperiment hat sich die Verwendung von frisch hergestellten, wäßrigen Lösungen, die i- p. oder i. v. gegeben werden, als zweckmäßig erwiesen.

Die erfindungsgemäß eingesetzten Verbindungen sind wirksam gegen verschiedene Leukämieformen sowie maligne Neoplasmen wie Lungencarcinom, Coloncarcinom, Melanome, Ependymoblastom und Sarcome. Es hat sich gezeigt, daß in einigen Fällen eine deutliche Überlegenheit gegenüber Cyclophosphamid und Fluoruracil festzustellen ist.

Eine Kombinationstherapie in Verbindung mit anderen Cytostatica wie Derivaten des Stickstofflosts oder auch Fluoruracil ist möglich.

Ganz allgemein gilt für die im Rahmen der Erfindung eingesetzten Verbindungen der allgemeinen Formel I mit einem Rest Z, daß dieser Rest Z wenigstens unter Normalbedingungen oder wenigstens unter Kühlung keine oder keine wesentliche Reaktivität mit den Epoxidgruppen des beziehungsweise der Glycidylsubstituenten am Ringsystem der allgemeinen Formel I zeigen beziehungsweise zeigen soll.

Auf diese Weise ist sichergestellt, daß die erfindungsgemäß verwendeten Wirkstoffe hinreichend lagerbeständig sind und keine unerwünschte Umsetzung unter Vernichtung der Epoxidgruppierungen stattfindet. Diese Regel ist insbesondere auch bei der Auswahl eventuell vorliegender Substituenten an dem Rest R zu berücksichtigen.

Beispiele für den Rest Z der erfindungsgemäß eingesetzten Verbindungen der allgemeinen Formel I mit cytostatischer Wirksamkeit sind die folgenden:

Methyl, Ethyl, Propyl, Butyl, Pentyl, Hexyl, die entsprechenden isomeren Reste wie Isopropyl, Isobutyl, tert.-Butyl, Isopentyl, entsprechende ungesättigte, insbesondere olefinisch ungesättigte Reste wie Vinyl, Allyl, Butenyl, Phenyl, Benzyl, Xylyl, Trimethylphenyl, Isopropylphenyl, Naphthyl, Cyclopentyl, Cyclohexyl, die entsprechenden mit 1 bis 3 Alkyl- beziehungsweise Alkenylresten substituierten cycloaliphatischen Reste, wobei die Alkyl- beziehungsweise Alkenylsubstituenten, vorzugsweise 1 bis 4 C-Atome aufweisen, 2,3-Dihydroxypropyl, 2-Hydroxy-3-diethylamino-propyl, 2-Hydroxy-3-dimethyl-amino-propyl, 2-Hydroxy-3-(dihydroxyethylamino)-propyl, 2-Hydroxy-3-morpholino-propyl, 2-Hydroxy-3-phenoxypropyl, 2-Hydroxy-3-methoxy-propyl, 2-Hydroxy-3-ethoxy-propyl, 2-Hydroxy-3-propoxy-propyl, 2-Hydroxy-3-acetoxy-propyl, 2-Hydroxy-3-propyloxy-propyl, 2-Hydroxy-3-butyloxy-propyl, 3-Hydroxy-2-acetoxy-propyl, 3-Hydroxy-2-propyloxy-propyl, 3-Hydroxy-2-butyloxy-propyl, 2-Hydroxy-3-chlor-propyl und 2-Hydroxy-3-brom-propyl.

Zusätzliche Beispiele für die in den Rahmen der Erfindung fallenden Möglichkeiten für R sind die folgenden Reste:

Halogenalkyl, Hydroxy-alkylthiopropyl, 2-Hydroxy-3-methylaminopropyl, 2-Hydroxy-3-ethylamino-propyl, 2-Hydroxy-3-benzyloxy-propyl und 2-Hydroxy-3-hydroxy-propyloxy-propyl.

Weitere Möglichkeiten für den Rest R sind

2-Hydroxy-3-methylthio-propyl, 2-Hydroxy-3-butyl-thio-propyl, 2-Hydroxy-3-phenylthio-propyl, 2-Hydroxy-3-acetthio-propyl und 2-Hydroxy-3-octylthio-propyl.

Reaktanten für die Umwandlung einer Glycidylgruppe im Triglycidylurazol unter Ausbildung eines substituierten Restes Z im Sinne der Erfindung sind ganz allgemein Alkanole wie

Methanol, Ethanol, n-Propanol, Isopropanol, n-Butanol, Isobutanol, sec.-Butanol, tert.-Butanol, 1-Pentanol, 2-Pentanol, 3-Pentanol, 2-Methyl-1-butanol, 3-Methyl-1-butanol, 2-Ethyl-2-butanol, 3-Methyl-2-butanol, 2,2-Dimethyl-1-propanol, 1-Hexanol, 2-Ethyl-1-butanol, 4-Methyl-1-pentanol, 4-Methyl-2-pentanol, 2-Methyl-1-pentanol.

Als ungesättigte Alkohole kommen beispielsweise in Betracht:

2-Buten-1-ol, 2-Propin-1-ol, Allylalkohol, Crotylalkohol, 3-Buten-2-ol, 2-Buten-1-ol und 3-Butin-2-ol.

Beispiele für mehrwertige Alkohole sind

Ethylenglykol, Propandiol-1,2, Propandiol-1,3, Butandiol-1,4, Butandiol-1,2, Butandiol-2,3, Butandiol-1,3, 2-Butendiol-1,4, 2-Butin-1,4-diol, 1,5-Pentandiol, 2-Methyl-1,4-butandiol, 2,2-Dimethyl-1,3-propandiol, Hexandiol, 2,5-Dimethyl-3-hexin-2,5-diol.

Beispiele für Thiole sind in diesem Zusammenhang

Methanthiol, Ethanthiol, 1-Propanthiol, 2-Propanthiol, 2-Methyl-2-propanthiol, 2-Butanthiol, 2-Methyl-1-propanthiol, 1-Butanthiol, 1-Pentanthiol, 1-Hexanthiol sowie 1,2-Ethanthiol, 2,2-Propanthiol, Benzenethiol, p-Benzenedithiol, Pyridin-2-thiol und Thiophen-2-thiol.

Die aus solchen Mercaptoresten erhaltenen Sulfoxidverbindungen fallen in den Rahmen der Erfindung. Beispiele für Carbonsäuren sind insbesondere Essigsäure, Propinsäure, n-Buttersäure, n-Valeriansäure, Caprinsäure, Önanthsäure, Isobuttersäure, 3-Methylbutansäure, 2,2-Dimethylpropansäure, 2-Methylbutansäure, 2-Ethylbutansäure, 2-Ethylhexansäure. Ungesättigte Säuren sind beispielsweise Propensäure, 2-Methylpropensäure, 3-Methylpropensäure, 2,3-Dimethylpropensäure, Hexadiensäure, Propiolsäure. Beispiele für aromatische Säuren sind Benzoesäure, und die entsprechenden mit Methyl- oder Ethylresten substituierten Säuren. Als phenolische Verbindungen kommen beispielsweise in Betracht: Phenol, $\alpha$-beziehungsweise $\beta$-Naphthol, Kresole, Xylenole, Chlorphenole, Chlorkresole, Chlorxylenole, Methylphenole mit gegebenenfalls mehr als einer Methylgruppe, zum Beispiel 2,3,4-Trimethylphenol, Ethylphenole, Propylphenole oder Butylphenole. Beispiele für die Umsetzung der Glycidylgruppe mit Aminen sind:

Methylamin, Dimethylamin, Ethylamin, Diethylamin, n-Propylamin, Di-n-propylamin, Iso-propylamin, Di-iso-propylamin, n-Butylamin, Di-n-butylamin, sec.-Butylamin, Di-sec.-butylamin, Iso-butylamin, Di-iso-butylamin, tert.-Butylamin, n-Amylamin, Di-n-amylamin, sec.-n-Amylamin, Iso-amylamin, Di-iso-amylamin, Allylamin, Di-allylamin, Cyclohexylamin, N-Methylcyclohexylamin, Dicyclohexylamin, Cyclooctylamin.

Beispiele für cyclische Verbindungen mit Aminogruppen sind: Piperidin, Hexamethylenimin, Morpholin, Anilin, $\alpha$- beziehungsweise $\beta$-Naphthylamin.

Die erfindungsgemäß beschriebenen Polyglycidyl-substituierten Urazole liegen in den Arzneimittelgemischen gemäß der Erfindung üblicherweise in Konzentrationen bis zu etwa 20 Gewichtsprozent — bezogen auf die Arzneimittelmischung — vor. Geeignet ist beispielsweise der Bereich von 0,05 bis 10 Gewichtsprozent, insbesondere ein Bereich von 0,05 bis 5 Gewichtsprozent.

Die Prozentangaben der nachfolgenden Beispiele beziehen sich jeweils auf Gewichtsprozent, so weit nicht im Einzelfall andere Angaben gemacht sind.

## Beispiele

### Beispiel 1

15,2 (0,15 Mol) Urazol wurden mit 0,4 g Tetramethylammoniumbromid und 0,5 g Benzalkon A (Mischung aus Alkylbenzyldimethylammoniumchloriden) in 416 g (4,5 Mol) Epi-Chlorhydrin 3 Stunden unter Rückfluß zum Sieden erhitzt. Nach dem Abkühlen wurde die Lösung mit 28,8 g (0,72 Mol) gepulvertem Natriumhydroxid versetzt und 6 Stunden bei 45° C gerührt. Anschließend wurde vom Niederschlag abgesaugt, die Lösung unter vermindertem Druck bei 40° C zur Trockne gedampft, der Rückstand in wenig Methylenchlorid gelöst und an Kieselgel (Merck) säulenchromatographiert (Elueirungsmittel), Methylenchlorid/Methanol (95 : 5).

Die einzelnen Fraktionen wurden nach DC-Kontrolle vereinigt. Die zweite Sammelfraktion (Substanz mit zweithöchsten $R_F$-Werten) ergab nach dem Eindampfen 1,5 g 1,2,4-Triglycidylurazol, das nach dem Umkristallisieren aus Ethylacetat unverändert bei 93° bis 94° C schmolz.

Epoxidzahl
gefunden: 17,1
berechnet: 17,8

Elementaranalyse und Massenspektrum stützen die Struktur.

### Beispiel 2

1,53 g (0,01 Mol) 4-Phenylurazol (Darstellung gemäß Org. Syntheses Vol. 51, S. 121 (1971)), 0,08 g Benzalkon A, 0,08 g Tetraethylammoniumbromid und 50 g Epi-Chlorhydrin werden 4,5 Stunden bei 60° C gerührt. Nach Abkühlen auf Raumtemperatur und Zugabe von 20 g Molekularsieb 4 Å werden 0,88 g (0,024 Mol) NaOH in 1 ml $H_2O$ zugetropft und 1,5 Stunden bei 45° C nachgerührt, dann abgesaugt und im Vakuum bei 40° C eingeengt.

| | |
|---|---|
| Auswaage: | 2,75 g |
| % EpO: | 8,9 |

Das rohe Reaktionsprodukt wird säulenchromatographisch getrennt.

| | |
|---|---|
| Säule: | |
| Höhe: | 40 cm, |
| Durchmesser: | 4 cm |
| Füllmittel: | Kieselgel 60 (Merck) |
| Laufmittel: | Methylenchlorid: Essigester: Methanol 3 : 2 : 1 |

Die Zone mit dem $R_F$-Wert von 0,65 wird isoliert.
Man erhält 1,85 g 1,2-Diglycidyl-4-phenylurazol

% EpO: 11,9 (Theorie 12,1)

Das Massen-, IR- und NMR-Spektrum stützen die Struktur.

### Beispiel 3

Die nachfolgenden Versuche wurden durchgeführt nach Testvorschriften des National Cancer Institute Bethesda, Maryland 200014, veröffentlicht in »Cancer Chemotherapy Reports« Part. 3, September 1972, Vol. 3, Nr. 2. Als Wirksubstanz wurden die nach Beispiel 1 und 2 hergestellten Verbindungen verwendet. Die Substanz wurde als wäßrige, 1%ige Injektionslösung unmittelbar vor der Applikation frisch hergestellt.

Bei Mäusen wurde gemäß Protokoll 1200 (S. 91c) die Tumorart P 388 (Leukämie) i. p. mit $10^6$ Zellen/Maus gesetzt. Die mittlere Überlebensdauer der unbehandelten Tiere wird bestimmt.

In weiteren Versuchsgruppen wird entsprechend vorbehandelten Tieren der Wirkstoff appliziert.
Es wird eine signifikante Verlängerung der Lebensdauer der behandelten Testtiere gegenüber der mittleren Überlebensdauer der nicht mit dem Wirkstoff behandelten Tiere erzielt. Die Verlängerungsrate T/C in Abhängigkeit von der Dosierung des Wirkstoffes ist in der folgenden Tabelle zusammengestellt:

Tabelle zu Beispiel 3

| Beispiel | verabreichte Dosis mg/kg | T/C-Wert |
|---|---|---|
| 1 | 50 | 298 |
| | 25 | >260 |
| | 12,5 | 230 |
| | 6,25 | 180 |
| 2 | 200 | 200 |
| | 100 | 150 |
| | 50 | 120 |
| | 25 | 120 |

## Beispiel 4

### N-Dihydroxypropyl-N',N''-diglycidyl-urazol

5 g Triglycidylurazol (0,019 Mol) werden in 50 ml Wasser 3 Stunden bei 70°C gerührt. Die Lösung wird am Rotavapor eingeengt und am Hochvakuum getrocknet. Das farblose, ölige Rohprodukt (5,8 g) wird durch Säulenchromatographie gereinigt.

| | | |
|---|---|---|
| Säule: | 35 × 5 cm | |
| Füllmittel: | Kieselgel 60; 0,063 – 0,2 mm (Merck) | |
| Lösungsmittel: | Essigester | 2 Teile |
| | Methylenchlorid | 2 Teile |
| | Methanol | 1 Teil |

Man erhält die gewünschte Verbindung als farbloses Öl.

| | |
|---|---|
| Rf: | 0,43 in obigem Elutionsmittel auf Kieselgelplatten |
| Ausbeute: | 30 Gewichtsprozent, Gew.-% bezogen auf Rohprodukt |
| % Epoxid-Sauerstoff: | 11,02 (berechnet 11,14) |

IR, MS und $^1$H-NMR stützen die Struktur.

## Beispiel 5

### N-(2-Hydroxy-3-propionoxy-propyl)-N',N''-diglycidyl-urazol

5 g Triglycidylurazol (0,019 Mol) werden mit 14 g Propionsäure (0,19 Mol) in 100 ml absolutem Toluol 3 Stunden bei 100 bis 110°C unter Zusatz von 5 g Molekularsieb 4 Å gerührt.
Nach Filtration des Molsiebes wird die Lösung eingeengt. Der Rückstand wird in Methylenchlorid gelöst und 2× mit 50 ml 10%iger Soda-Lösung ausgeschüttelt. Die über Natriumsulfat getrocknete Methylenchlorid-Phase wird eingeengt. Der erhaltene farblose, ölige Rückstand (5,2 g) wird durch Säulenchromatographie gereinigt.

| | |
|---|---|
| Säule: | 35 × 5 cm |
| Füllung: | Kieselgel 60 (Merck) 0,063—0,2 mm |
| Lösungsmittel: | Methylenchlorid + 5% Methanol |

Die gewünschte Verbindung wird als farbloses Öl isoliert.

| | |
|---|---|
| Ausbeute: | 21 Gewichtsprozent |
| Rf: | 0,3 im obengenannten Fließmittel auf Kieselgel-Platten |
| % Epoxid-Sauerstoff: | 9,5 (berechnet 9,3) |

IR und $^1H$-NMR stützen die Struktur.

## Beispiel 6

### N-(2-Hydroxy-3-morpholin-N-yl-propyl)-N',N''-diglycidylurazol

5 g Triglycidylurazol (0,019 Mol) werden mit 2 ml Morpholin (0,022 Mol) in 100 ml absolutem Isopropanol 3 Stunden bei 50°C gerührt. Die Lösung wird eingeengt und am Hochvakuum getrocknet. Das gelbliche, ölige Rohprodukt (7,4 g) wird säulenchromatographisch gereinigt.

| | |
|---|---|
| Säule: | 35 × 5 cm |
| Füllung: | Kieselgel 60 (Merck) 0,063—0,2 mm |
| Lösungsmittel: | Methylenchlorid + 20% Methanol |

Die gewünschte Verbindung wird als farbloses Öl isoliert.

| | |
|---|---|
| Ausbeute: | 51 Gewichtsprozent |
| Rf: | 0,72 im obengenannten Fließmittel auf Kieselgel-Platten |
| % Epoxid-Sauerstoff: | 9,0 (berechnet 9,0) |

IR und $^1H$-NMR stützen die Struktur.

Elementaranalyse:

| | | | |
|---|---|---|---|
| gef. | C: 50,7% | H: 6,2% | N: 15,5% |
| ber. | C: 50,55% | H: 6,79% | N: 15,72% |

## Beispiel 7

### 1,2-Diglycidyl-4-methyl-urazol

8,5 g (0,074 Mol) 4-Methylurazol (Darstellung gemäß Literatur: R.C. Cookson, S.S. Gupte et al.; Org. Synth. 51 (1971), S. 121) werden mit 2% Tetraethylammoniumbromid in 230 ml Epichlorhydrin (2,95 Mol) 4 Stunden bei 80°C gerührt. Die abgekühlte Lösung wird nach Zusatz von 65 g Molekularsieb 4 Å mit 24 g 50%iger Natronlauge (0,3 Mol) 3 Stunden bei 40—50°C gerührt. Nach Absaugen wird das Filtrat eingedampft und der hellgelbe feste Rückstand aus Methanol umkristallisiert. Die erhaltenen 4,5 g (27% d. Th.) weißer Kristalle mit einem Epoxid-Sauerstoff-Gehalt von 14% (ber. 14,1%) schmelzen bei 90°C.

IR, $^1H$-NMR und MS stützen die Struktur.

## Beispiel 8

### 1,2-Diglycidyl-4-butyl-urazol

Es wird wie im Beispiel 7 gearbeitet, jedoch 4-Butyl-urazol als Ausgangsmaterial eingesetzt. Die Reinigung des Rohproduktes erfolgt durch Säulenchromatographie.

| | |
|---|---|
| Säule: | 40 × 5 cm |
| Füllung: | Kieselgel 60 (Merck) 0,063—0,2 mm |
| Fließmittel: | Methylenchlorid + 5% Methanol |

Es werden 36 Gewichtsprozent reines Produkt isoliert.

| | |
|---|---|
| Rf: | 0,65 im obengenannten Fließmittel auf Kieselgel-Platten |
| % Epoxid-Sauerstoff: | 11,7 (berechnet 11,9) |
| Fp der weißen Kristalle: | 49—52° C |

IR, $^1$H-NMR stützen die Struktur.

Beispiel 9

Die nachfolgenden Ergebnisse zeigen die Wirksamkeit der gemäß Beispiel 4 bis 8 hergestellten Verbindungen in der Bekämpfung der Leukämie (Tumor P 388) und wurden, wie im Beispiel 3 erläutert, durchgeführt. In der nachstehenden Tabelle sind in Abhängigkeit von gemäß Beispiel 4 bis 8 hergestellten Wirkstoffen die bei variabler Dosierung (mg Wirksubstanz/kg Maus) gefundenen T/C-Werte wiedergegeben.

Tabelle

| Beispiel | mg/kg | T/C |
|---|---|---|
| Nr. 4 | 100 | >300 |
| | 50 | 278 |
| Nr. 5 | 100 | 250 |
| | 50 | 230 |
| Nr. 6 | 200 | 230 |
| | 100 | 160 |
| Nr. 7 | 100 | >300 |
| | 50 | 210 |
| Nr. 8 | 200 | 243 |
| | 100 | 180 |
| | 50 | 153 |

**Patentansprüche für die Vertragsstaaten BE, LU, NL, SE**

1. N-substituierte Polyglycidyl-urazolverbindungen der allgemeinen Formel I

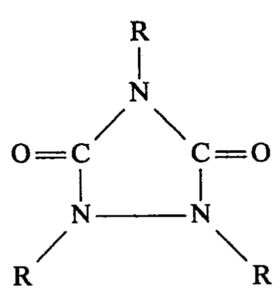

(I)

in der die Reste R einen Glycidylrest der allgemeinen Formel II

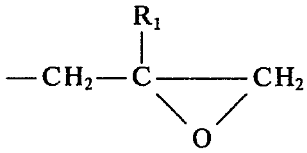

(II)

bedeuten, worin $R_1$ Wasserstoff oder einen Alkylrest mit 1 bis 4 C-Atomen ist, oder zwei der Reste R einen Glycidylrest der allgemeinen Formel II bedeuten und dann der verbleibende Rest R ein Rest Z ist, der 1 bis 15 C-Atome, Wasserstoff und gegebenenfalls bis zu 3 Heteroatome enthält.

2. Polyglycidyl-urazolverbindungen der allgemeinen Formel I, dadurch gekennzeichnet, daß die drei Reste R einen Glycidylrest der allgemeinen Formel II bedeuten, in dem $R_1$ Wasserstoff ist.

3. Polyglycidyl-urazolverbindungen nach Anspruch 1, dadurch gekennzeichnet, daß zwei der Reste R einen Glycidylrest der Formel II bedeuten, in dem $R_1$ vorzugsweise Wasserstoff ist und Z als Heteroatome O, N, S, und/oder P enthält.

4. Polyglycidyl-urazolverbindungen nach Ansprüchen 1 und 3, dadurch gekennzeichnet, daß Z Alkyl, Aryl, Aralkyl, Alkaryl oder Cycloalkyl bedeutet, welche Reste gewünschtenfalls auch heterocyclischer Natur, ungesättigt und/oder mit wenigstens einem der folgenden Substituenten substituiert sein können: Halogen, Hydroxyl, Amino, N-substituiertes Amino, Mercapto, Alkylmercapto, Arylmercapto, Alkylsulfoxy, Arylsulfoxy, Alkoxy, Aryloxy und heterocyclischer Rest.

5. Polyglycidyl-urazolverbindungen nach Ansprüchen 1, 3 und 4, dadurch gekennzeichnet, daß der Rest Z nicht mehr als 12 Kohlenstoffatome und insbesondere nicht mehr als 8 Kohlenstoffatome aufweist.

6. Polyglycidyl-urazolverbindungen nach Ansprüchen 1, 3 bis 5, dadurch gekennzeichnet, daß der Rest Z ein gegebenenfalls substituierter Alkylrest mit bis zu 10 C-Atomen, vorzugsweise mit bis zu 6 C-Atomen, insbesondere mit bis zu 3 C-Atomen ist.

7. Polyglycidyl-urazolverbindungen nach Ansprüchen 1, 3 bis 6, dadurch gekennzeichnet, daß der Rest Z ein mit wenigstens einer Hydroxylgruppe substituierter Alkylrest ist.

8. Polyglycidyl-urazolverbindungen nach Ansprüchen 1, 3 bis 7, dadurch gekennzeichnet, daß Z ein Hydroxyalkylrest ist, der wenigstens einen weiteren der folgenden Substituenten aufweist: Hydroxyl, Halogen, Amino, N-substituiertes Amino, Mercapto, Alkylmercapto, Arylmercapto, Alkylsulfoxy, Arylsulfoxy, Alkoxy, Aryloxy und Acyl.

9. Polyglycidyl-urazolverbindungen nach Ansprüchen 1, 3 bis 8, dadurch gekennzeichnet, daß Z unsubstituiertes oder monohydroxyliertes Alkyl mit 1 bis 6 C-Atomen oder disubstituiertes Alkyl mit 3 bis 7 C-Atomen aus der Gruppe Dihydroxyalkyl, Halogenhydroxyalkyl, N-substituiertes Aminohydroxyalkyl, Alkoxyhydroxyalkyl, Alkylmercaptohydroxyalkyl, Alkylsulfoxyhydroxyalkyl und Acyloxyhydroxyalkyl ist.

10. Polyglycidyl-urazolverbindungen nach Ansprüchen 1, 3 bis 9, dadurch gekennzeichnet, daß Z ein disubstituierter $C_3$-Alkylrest ist, der die Hydroxygruppe in 2- oder 3-Stellung und den anderen Substituenten in der anderen der beiden genannten Stellungen am $C_3$-Alkylrest aufweist.

11. Verfahren zur Herstellung von N-substituierten Polyglycidyl-urazolverbindungen nach Ansprüchen 1 bis 10, dadurch gekennzeichnet, daß man die drei Glycidylreste der allgemeinen Formel II in Urazol in N-Substitution einführt, gewünschtenfalls die Triglycidyl-urazolverbindung einer partiellen Umsetzung mit Wasser, Alkoholen, Verbindungen mit einer primären und/oder sekundären Aminogruppe, Mercaptanen, Schwefelwasserstoff, Carbonsäuren, Halogenwasserstoff oder Wasserstoff beziehungsweise Wasserstoff liefernden Verbindungen unterwirft und gewünschtenfalls gebildete Mercaptoverbindungen zu entsprechenden Sulfoxyverbindungen umwandelt und daß man in eine mit dem Rest Z mono-N-substituierte Urazolverbindung die beiden Glycidylreste der allgemeinen Formel II in N-Substitution einführt.

12. Verfahren nach Anspruch 11, dadurch gekennzeichnet, daß zur Einführung der Glycidylreste der allgemeinen Formel II Urazol beziehungsweise eine mit dem Rest Z monosubstituierte Urazolverbindung mit Epi-Halohydrinen umgesetzt wird und die Halohydringruppen anschließend dehydrohalogeniert werden beziehungsweise die eingesetzte Urazolverbindung zunächst mit Allylhalogeniden umgesetzt wird und die Allylgruppen anschließend epoxidiert werden.

13. Verfahren nach Ansprüchen 11 und 12, dadurch gekennzeichnet, daß die Umsetzung der Urazolverbindungen, mit Epi-Halogydrinen beziehungsweise Allylhalogenid in Gegenwart von Phasentransfer-Katalysatoren vorgenommen wird.

14. N-substituierte Polyglycidyl-urazolverbindungen gemäß Ansprüchen 1 bis 10 zur Anwendung als cytostatische Mittel.

**Patentansprüche für die Vertragsstaaten CH, DE, FR, GB, IT, LI**

1. N-substituierte Polyglycidyl-urazolverbindungen der allgemeinen Formel I

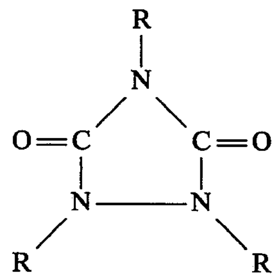

(I)

in der die Reste R einen Glycidylrest der allgemeinen Formel II

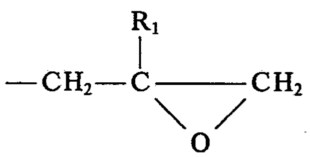

(II)

bedeuten, worin $R_1$ Wasserstoff oder einen Alkylrest mit 1 bis 4 C-Atomen ist, oder zwei der Reste R einen Glycidylrest der allgemeinen Formel II bedeuten und dann der verbleibende Rest R ein Rest Z ist, der 1 bis 15 C-Atome, Wasserstoff und gegebenenfalls bis zu 3 Heteroatome enthält, zur Anwendung als cytostatische Mittel.

2. N-substituierte Polyglycidyl-urazolverbindungen nach Anspruch 1, bei denen die 3 Reste R einen Glycidylrest der allgemeinen Formel II bedeuten, in dem $R_1$ Wasserstoff ist zur Anwendung als cytostatische Mittel.

3. N-substituierte Polyglycidyl-urazolverbindungen der allgemeinen Formel

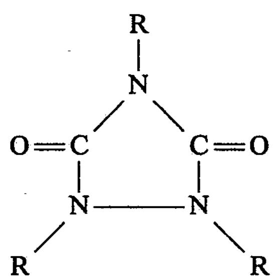

(I)

in der die Reste R einen Glycidylrest der allgemeinen Formel II

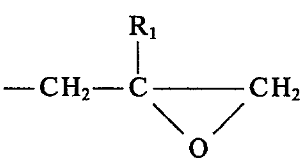

(II)

bedeuten, worin $R_1$ Wasserstoff oder einen Alkylrest mit 1 bis 4 C-Atomen ist, oder zwei der Reste R einen Glycidylrest der allgemeinen Formel II bedeuten und dann der verbleibende Rest R ein Rest Z ist, der 1 bis 15 C-Atome, Wasserstoff und gegebenenfalls bis zu 3 Heteroatome enthält, wobei Verbindungen ausgenommen sind, die 3 Glycidylreste enthalten, in denen $R_1$ Wasserstoff oder die Methylgruppe ist.

4. Polyglycidyl-urazolverbindungen nach Anspruch 3, dadurch gekennzeichnet, daß 2 der Reste R einen Glycidylrest der Formel II bedeuten, in dem $R_1$ vorzugsweise Wasserstoff ist und Z als Heteroatome O, N, S und/oder P enthält.

5. Polyglycidyl-urazolverbindungen nach Anspruch 3, dadurch gekennzeichnet, daß Z Alkyl, Aryl, Aralkyl, Alkaryl oder Cycloalkyl bedeutet, welche Reste gewünschtenfalls auch heterocyclischer Natur, ungesättigt und/oder mit wenigstens einem der folgenden Substituenten substituiert sein können: Halogen, Hydroxyl, Amino, N-substituiertes Amino, Mercapto, Alkylmercapto, Arylmercapto, Alkylsulfoxy, Arylsulfoxy, Alkoxy, Aryloxy, Acyloxy und heterocyclischer Rest.

6. Polyglycidyl-urazolverbindungen nach Ansprüchen 3 und 4, dadurch gekennzeichnet, daß der Rest Z nicht mehr als 12 Kohlenstoffatome und insbesondere nicht mehr als 8 Kohlenstoffatome aufweist.

7. Polyglycidyl-urazolverbindungen nach Ansprüchen 3 bis 5, dadurch gekennzeichnet, daß der Rest Z ein gegebenenfalls substituierter Alkylrest mit bis zu 10 C-Atomen, vorzugsweise mit bis zu 6 C-Atomen, insbesondere mit bis zu 3 C-Atomen ist.

8. Polyglycidyl-urazolverbindungen nach Ansprüchen 3 bis 6, dadurch gekennzeichnet, daß der Rest Z ein mit wenigstens einer Hydroxylgruppe substituierter Alkylrest ist.

9. Polyglycidyl-urazolverbindungen nach Ansprüchen 3 bis 7, dadurch gekennzeichnet, daß Z ein Hydroxyalkylrest ist, der wenigstens einen weiteren der folgenden Substituenten aufweist: Hydroxyl, Halogen, Amino, N-substituiertes Amino, Mercapto, Alkylmercapto, Arylmercapto, Alkylsulfoxy, Arylsulfoxy, Alkoxy, Aryloxy und Acyl.

10. Polyglycidyl-urazolverbindungen nach Ansprüchen 3 bis 8, dadurch gekennzeichnet, daß Z unsubstituiertes oder monohydroxyliertes Alkyl mit 1 bis 6 C-Atomen oder disubstituiertes Alkyl mit 3 bis 7 C-Atomen aus der Gruppe Dihydroxyalkyl, Halogenhydroxyalkyl, N-substituiertes Aminohydroxyalkyl, Alkoxyhydroxyalkyl, Alkylmercaptohydroxyalkyl, Alkylsulfoxyhydroxyalkyl und Acyloxyhydroxyalkyl ist.

11. Polyglycidyl-urazolverbindungen nach Ansprüchen 3 bis 9, dadurch gekennzeichnet, daß Z ein disubstituierter $C_3$-Alkylrest ist, der die Hydroxygruppe in 2- oder 3-Stellung und den anderen Substituenten in der anderen der beiden genannten Stellungen am $C_3$-Alkylrest aufweist.

12. Verfahren zur Herstellung von N-substituierten Polyglycidyl-urazolverbindungen nach den Ansprüchen 3 bis 11, dadurch gekennzeichnet, daß man Triglycidylurazol einer partiellen Umsetzung mit Wasser, Alkoholen, Verbindungen mit einer primären und/oder sekundären Aminogruppe, Mercaptanen, Schwefelwasserstoff, Carbonsäure, Halogenwasserstoff oder Wasserstoff beziehungsweise Wasserstoff liefernden Verbindungen unterwirft und gewünschtenfalls gebildete Mercaptoverbindungen zu entsprechenden Sulfoxyverbindungen umwandelt oder daß man in eine mit dem Rest Z mono-N-substituierte Urazolverbindung die beiden Glycidylreste der allgemeinen Formel II in N-Substitution einführt.

13. Verfahren nach Anspruch 12, dadurch gekennzeichnet, daß zur Einführung der Glycidylreste der allgemeinen Formel II die eingesetzte Urazolverbindung zunächst mit Allylhalogeniden umgesetzt wird und die Allylgruppen anschließend epoxidiert werden.

14. Verfahren nach Ansprüchen 12 und 13, dadurch gekennzeichnet, daß die Umsetzung der Urazolverbindungen mit Epi-Halohydrinen beziehungsweise Allylhalogenid in Gegenwart von Phasentransfer-Katalysatoren vorgenommen wird.

**Patentansprüche für den Vertragsstaat: AT**

1. Verfahren zur Herstellung von N-substituierten Polyglycidyl-urazolverbindungen der allgemeinen Formel I

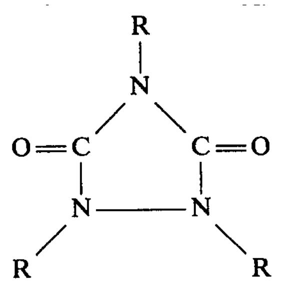

(I)

in der die Reste R einen Glycidylrest der allgemeinen Formel II

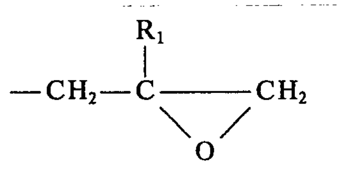

(II)

bedeuten, worin $R_1$ Wasserstoff oder einen Alkylrest mit 1 bis 4 C-Atomen ist, oder zwei der Reste R einen Glycidylrest der allgemeinen Formel II bedeuten und dann der verbleibende Rest R ein Rest Z ist, der 1 bis 15 C-Atome, Wasserstoff und gegebenenfalls bis zu 3 Heteroatome enthält.

2. Verfahren zur Herstellung von Polyglycidyl-urazolverbindungen der allgemeinen Formel I, dadurch gekennzeichnet, daß die drei Reste R einen Glycidylrest der allgemeinen Formel II bedeuten, in dem $R_1$ Wasserstoff ist.

3. Verfahren zur Herstellung von Polyglycidyl-urazolverbindungen nach Anspruch 1, dadurch gekennzeichnet, daß zwei der Reste R einen Glycidylrest der Formel II bedeuten, in dem $R_1$ vorzugsweise Wasserstoff ist und Z als Heteroatome O, N, S und/oder P enthält.

4. Verfahren zur Herstellung von Polyglycidyl-urazolverbindungen nach Ansprüchen 1 und 3, dadurch gekennzeichnet, daß Z Alkyl, Aryl, Aralkyl, Alkaryl oder Cycloalkyl bedeutet, welche Reste gewünschtenfalls auch heterocyclischer Natur, ungesättigt und/oder mit wenigstens einem der folgenden Substituenten substituiert sein können: Halogen, Hydroxyl, Amino, N-substituiertes Amino, Mercapto, Alkylmercapto, Arylmercapto, Alkylsulfoxy, Arylsulfoxy, Alkoxy, Aryloxy, Acyloxy und heterocyclischer Rest.

5. Verfahren zur Herstellung von Polyglycidyl-urazolverbindungen nach Ansprüchen 1, 3 und 4, dadurch gekennzeichnet, daß der Rest Z nicht mehr als 12 Kohlenstoffatome und insbesondere nicht mehr als 8 Kohlenstoffatome aufweist.

6. Verfahren zur Herstellung von Polyglycidyl-urazolverbindungen nach Ansprüchen 1, 3 bis 5, dadurch gekennzeichnet, daß der Rest Z ein gegebenenfalls substituierter Alkylrest mit bis zu 10 C-Atomen, vorzugsweise mit bis zu 6 C-Atomen, insbesondere mit bis zu 3 C-Atomen ist.

7. Verfahren zur Herstellung von Polyglycidyl-urazolverbindungen nach Ansprüchen 1, 3 bis 6, dadurch gekennzeichnet, daß der Rest Z ein mit wenigstens einer Hydroxylgruppe substituierter Alkylrest ist.

8. Verfahren zur Herstellung von Polyglycidyl-urazolverbindungen nach Ansprüchen 1, 3 bis 7, dadurch gekennzeichnet, daß Z ein Hydroxyalkylrest ist, der wenigstens einen weiteren der folgenden Substituenten aufweist: Hydroxyl, Halogen, Amino, N-substituiertes Amino, Mercapto, Alkylmercapto, Arylmercapto, Alkylsulfoxy, Arylsulfoxy, Alkoxy, Aryloxy und Acyl.

9. Verfahren zur Herstellung von Polyglycidyl-urazolverbindungen nach Ansprüchen 1, 3 bis 8, dadurch gekennzeichnet, daß Z unsubstituiertes oder monohydroxyliertes Alkyl mit 1 bis 6 C-Atomen oder disubstituiertes Alkyl mit 3 bis 7 C-Atomen aus der Gruppe Dihydroxyalkyl, Halogenhydroxyalkyl, N-substituiertes Aminohydroxyalkyl, Alkoxyhydroxyalkyl, Alkylmercaptohydroxyalkyl, Alkylsulfoxyhydroxyalkyl und Acyloxyhydroxyalkyl ist.

10. Verfahren zur Herstellung von Polyglycidyl-urazolverbindungen nach Ansprüchen 1, 3 bis 9, dadurch gekennzeichnet, daß Z ein disubstituierter $C_3$-Alkylrest ist, der die Hydroxygruppe in 2- oder 3-Stellung und den anderen Substituenten in der anderen der beiden genannten Stellungen am $C_3$-Alkylrest aufweist.

11. Verfahren zur Herstellung von N-substituierten Polyglycidyl-urazolverbindungen nach Ansprüchen 1 bis 10, dadurch gekennzeichnet, daß man die drei Glycidylreste der allgemeinen Formel II in Urazol in N-Substitution einführt, gewünschtenfalls die Triglycidyl-urazolverbindung einer partiellen Umsetzung mit Wasser, Alkoholen, Verbindungen mit einer primären und/oder sekundären Aminogruppe, Mercaptanen, Schwefelwasserstoff, Carbonsäuren, Halogenwasserstoff oder Wasserstoff beziehungsweise Wasserstoff liefernden Verbindungen unterwirft und gewünschtenfalls gebildete Mercaptoverbindungen zu entsprechenden Sulfoxyverbindungen umwandelt und daß man in eine mit dem Rest Z mono-N-substituierte Urazolverbindung die beiden Glycidylreste der allgemeinen Formel II in N-Substitution einführt.

12. Verfahren nach Anspruch 11, dadurch gekennzeichnet, daß zur Einführung der Glycidylreste der allgemeinen Formel II Urazol beziehungsweise eine mit dem Rest Z monosubstituierte Urazolverbindung mit Epi-Halogydrinen umgesetzt wird und die Halohydringruppen anschließend dehydrohalogeniert werden beziehungsweise die eingesetzte Urazolverbindung zunächst mit Allylhalogeniden umgesetzt wird und die Allylgruppen anschließend epoxidiert werden.

13. Verfahren nach Ansprüchen 11 und 12, dadurch gekennzeichnet, daß die Umsetzung der Urazolverbindungen, mit Epi-Halohydrinen beziehungsweise Allylhalogenid in Gegenwart von Phasentransfer-Katalysatoren vorgenommen wird.

14. N-substituierte Polyglycidyl-urazolverbindungen gemäß Ansprüchen 1 bis 10 zur Anwendung als cytostatische Mittel.

**Claims for the contracting States BE, LU, NL, SE**

1. N-substituted polyglycidyl urazole compounds corresponding to the following general formula

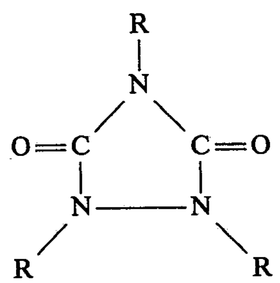

(I)

in which the radicals R represent a glycidyl radical corresponding to the following general formula

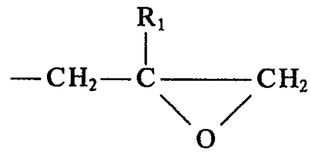

(II)

in which $R_1$ is hydrogen or a $C_1-C_4$ alkyl radical, or two of the radicals R represent a glycidyl radical corresponding to general formula (II) and the remaining radical R is a radical Z containing from 1 to 15 C-atoms, hydrogen and optionally up to 3 hetero atoms.

2. Polyglycidyl urazole compounds corresponding to general formula (I), characterized in that the three radicals R represent a glycidyl radical corresponding to general formula (II) in which $R_1$ is hydrogen.

3. Polyglycidyl urazole compounds as claimed in Claim 1, characterized in that two of the radicals R represent a glycidyl radical corresponding to general formula (II) in which $R_1$ is preferably hydrogen and Z contains O, N, S and/or P as hetero atoms.

4. Polyglycidyl urazole compounds as claimed in Claims 1 and 3, characterized in that Z represents alkyl, aryl, aralkyl, alkaryl or cycloalkyl radicals which, if desired, may even be heterocyclic, unsaturated and/or substituted by at least one of the following substituents: halogen, hydroxyl, amino, N-substituted amino, mercapto, alkylmercapto, arylmercapto, alkylsulfoxy, aryl sulfoxy, alkoxy, aryloxy, acyloxy or a heterocyclic radical.

5. Polyglycidyl urazole compounds as claimed in Claims 1, 3 and 4, characterized in that the radical Z contains no more than 12 C-atoms and, in particular, no more than 8 C-atoms.

6. Polyglycidyl urazole compounds as claimed in Claims 1 and 3 to 5, characterized in that the radical Z is an optionally substituted alkyl radical containing up to 10 C-atoms, preferably up to 6 C-atoms and, more preferably, up to 3 C-atoms.

7. Polyglycidyl urazole compounds as claimed in Claims 1 and 3 to 6, characterized in that the radical Z is an alkyl radical substituted by at least one hydroxyl group.

8. Polyglycidyl urazole compounds as claimed in Claims 1 and 3 to 7, characterized in that Z is a hydroxyalkyl radical containing at least one other of the following substituents: hydroxyl, halogen, amino, N-substituted amino, mercapto, alkylmercapto, arylmercapto, alkylsulfoxy, arylsulfoxy, alkoxy, aryloxy and acyl.

9. Polyglycidyl urazole compounds as claimed in Claims 1 and 3 to 8, characterized in that Z represents unsubstituted or monohydroxylated alkyl containing from 1 to 6 C-atoms or disubstituted alkyl containing from 3 to 7 C-atoms from the group comprising dihydroxyalkyl, halogenhydroxyalkyl, N-substituted aminohydroxyalkyl, alkoxyhydroxyalkyl, alkylmercaptohydroxyalkyl and acyloxyhydroxyalkyl.

10. Polyglycidyl urazole compounds as claimed in Claims 1 and 3 to 9, characterized in that Z is a disubstituted $C_3$-alkyl radical which contains the hydroxy group in the 2- or 3-position and the other substituents in the other two positions mentioned on the $C_3$-alkyl radical.

11. A process for producing the N-substituted polyglycidyl urazole compounds claimed in Claims 1 to 10, characterized in that the three glycidyl radicals corresponding to general formula (II) are introduced into urazole in N-substitution, the triglycidyl urazole compounds is, if desired, subjected to a partial reaction with water, alcohols, compounds containing a primary and/or secondary amino group, mercaptans, hydrogen sulfide, carboxylic acids, hydrogen halide or hydrogen or hydrogen-yielding compounds and, if desired, the mercapto compounds formed are converted into corresponding sulfoxy compounds and in that the two glycidyl radicals corresponding to general formula (II) are introduced in N-substitution into a urazole compound mono-N-substituted by the radical Z.

12. A process as claimed in Claim 11, characterized in that, for introducing the glycidyl radicals corresponding to general formula (II), urazole or a urazole compound monosubstituted by the radical Z is reacted with epihalohydrins and the halohydrin groups are subsequently dehydrohalogenated or the urazole compound used is first reacted with allyl halides and the allyl groups subsequently epoxidized.

13. A process as claimed in Claims 11 and 12, characterized in that the reaction of the urazole compounds with epihalohydrins or allyl halide is carried out in the presence of phase transfer catalysts.

14. N-substituted polyglycidyl urazole compounds as claimed in Claims 1 to 10 for use as cytostatic agents.

**Claims for the contracting States: CH, DE, FR, GB, IT, LI**

1. N-substituted polyglycidyl urazole compounds corresponding to the following general formula

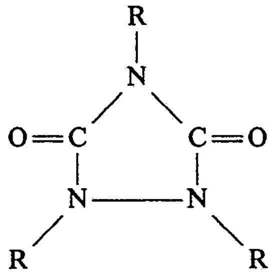

(I)

in which the radicals R represent a glycidyl radical corresponding to the following general formula

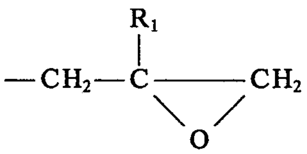

(II)

in which $R_1$ is hydrogen or a $C_1-C_4$ alkyl radical, or two of the radicals R represent a glycidyl radical corresponding to general formula (II) and the remaining radical R is a radical Z containing from 1 to 15 C-atoms, hydrogen and optinally up to 3 hetero atoms, for use as dytostatic agents.

2. N-substituted polyglycidyl urazole compounds as claimed in Claim 1 in which the three radicals R represent a glycidyl radical corresponding to general formula (II), in which $R_1$ is hydrogen, for use as dytostatic agents.

3. N-substituted polyglycidyl urazole compounds corresponding to the following general formula

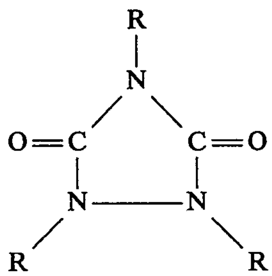

(I)

in which the radicals R represent a glycidyl radical corresponding to the following general formula

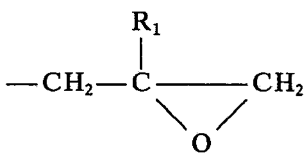

(II)

in which $R_1$ is hydrogen or a $C_1-C_4$ alkyl radical, or two of the radicals R represent a glycidyl radical corresponding to general formula (II) and the remaining radical R is a radical Z containing from 1 to 15 C-atoms, hydrogen and optionally up to 3 hetero atoms, excluding compounds containing three glycidyl radicals in which $R_1$ is hydrogen or the methyl group.

4. Polyglycidyl urazole compounds as claimed in Claim 3, characterzid in that two of the radicals R represent a glycidyl radical corresponding to formula (II) in which $R_1$ is preferably hydrogen and Z contains O, N, S and/or P as hetero atoms.

5. Polyglycidyl urazole compounds as claimed in Claim 3, characterized in that Z represents alkyl, aryl, aralkyl, alkaryl or cycloalkyl radicals which, if desired, may even be heterocyclic, unsaturated and/or substituted by at least one of the following substitutents: halogen, hydroxyl, amino, n-substituted amino, mercapto, alkylmercapto, arylmercapto, alkylsulfoxy, arylsulfoxy, alkoxy, aryloxy, acyloxy or a heterocyclic radical.

6. Polyglycidyl urazole compounds as claimed in Claims 3 and 4, characterized in that the radical Z contains no more than 12 carbon atoms and, more especially, no more than 8 carbon atoms.

7. Polyglycidyl urazole compounds as claimed in Claims 3 to 5, characterized in that the radical Z is an optionally substituted alkyl radical containing up to 10 C-atoms, preferably up to 6 C-atoms and more preferably up to 3 C-atoms.

8. Polyglycidyl urazole compounds as claimed in Claims 3 to 6, characterized in that the radical Z is an alkyl radical substituted by at least one hydroxyl group.

9. Polyglycidyl urazole compounds as claimed in Claims 3 to 7, characterized in that Z is a hydroxyalkyl radical containing at least one other of the following substituents: hydroxyl, halogen, amino, N-substituted amino, mercapto, alkylmercapto, arylmercapto, alkylsulfoxy, arylsulfoxy, alkoxy, aryloxy and acyl.

10. Polyglycidyl urazole compounds as claimed in Claims 3 to 8, characterized in that Z is unsubstituted or monohydroxylated $C_1-C_6$ alkyl or disubstituted $C_3-C_7$ alkyl from the group comprising dihydroxyalkyl, halogenhydroxyalkyl, N-substitued aminohydroxyalkyl, alkoxyhydroxyalkyl, alkylmercaptohydroxyalkyl, alkylsulfoxyhydroxyalkyl and acyloxyhydroxyalkyl.

11. Polyglycidyl urazole compounds as claimed in Claims 3 to 9, characterized in that Z is a disubstituted $C_3$-alkyl radical containing the hydroxy group in the 2- or 3-position and the other substitutent in the other of the two positions mentioned on the $C_3$-alkyl radical.

12. A process for producing the N-substituted polyglycidyl urazole compounds as claimed in Claims 3 to 11, characterized in that triglycidyl urazole is subjected to a partial reaction with water, alcohols, compounds containing a primary and/or secondary amino group, mercaptans, hydrogen sulfide, carboxylic acids, hydrogen halide or hydrogen or hydrogen-yielding compounds and, if desired, the mercapto compounds formed are converted into corresponding sulfoxy compounds or in that the two glycidyl radicals corresponding to general formula (II) are introduced in N-substitution into a urazole compound mono-N-substituted by the radical Z.

13. A process as claimed in Claim 12, characterized in that, for introducing the glycidyl radicals corresponding to general formula (II), the urazole compound used is first reacted with allyl halides and the allyl groups are subsequently epoxidized.

14. A process as claimed in Claims 12 and 13, characterized in that the reaction of the urazole compounds with epihalogydrins or allyl halide is carried out in the presence of phase transfer catalysts.

**Claims for the contracting State: AT**

1. A process for the production of N-substituted polyglycidyl urazole compounds corresponding to the following general formula

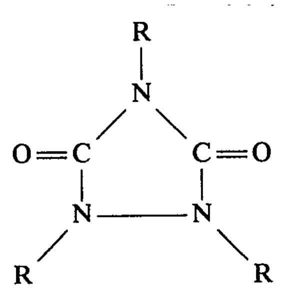

(I)

in which the radicals R represent a glycidyl radical corresponding to the following general formula

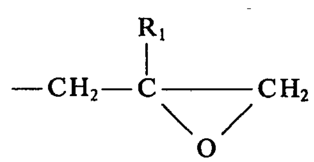

(II)

in which $R_1$ is hydrogen or a $C_1-C_4$ alkyl radical, or two of the radicals R represent a glycidyl radical corresponding to general formula (II) and the remaining radical R is a radical Z containing from 1 to 15 C-atoms, hydrogen and optionally up to 3 hetero atoms.

2. A process for the production of polyglycidyl urazole compounds corresponding to general formula (I), characterized in that the three radicals R represent a glycidyl radical corresponding to general formula (II) in which $R_1$ is hydrogen.

3. A process for the production of polyglycidyl urazole compounds as claimed in Claim 1, characterized in that two of the radicals R represent a glycidyl radical corresponding to general formula (II) in which $R_1$ is preferably hydrogen and Z contains O, N, S and/or P as hetero atoms.

4. A process for the production of polyglycidyl urazole compounds as claimed in Claims 1 and 3, characterized in that Z represents alkyl, aryl, aralkyl, alkaryl or cycloalkyl radicals which, if desired, may even be heterocyclic, unsaturated and/or substituted by at least one of the following substituents: halogen, hydroxyl, amino, N-substituted amino, mercapto, alkylmercapto, arylmercapto, alkylsulfoxy, arylsulfoxy, alkoxy, aryloxy, acyloxy or a heterocyclic radical.

5. A process for the production of polyglycidyl urazole compounds as claimed in Claims 1, 3 and 4, characterized in that the radical Z contains no more than 12 C-atoms and, in particular, no more than 8 C-atoms.

6. A process for the production of polyglycidyl urazole compounds as claimed in Claims 1 and 3 to 5, characterized in that the radical Z is an optionally substituted alkyl radical containing up to 10 C-atoms, preferably up to 6 C-atoms and, more preferably, up to 3 C-atoms.

7. A process for the production of polyglycidyl urazole compounds as claimed in Claims 1 and 3 to 6, characterized in that the radical Z is an alkyl radical substituted by at least one hydroxylgroup.

8. A process for the production of polyglycidyl urazole compounds as claimed in Claims 1 and 3 to 7, characterized in that Z is a hydroxyalkyl radical containing at least one other of the following substituents: hydroxyl, halogen, amino, N-substituted amino, mercapto, alkylmercapto, arylmercapto, alkylsulfoxy, arylsulfoxy, alkoxy, aryloxy and acyl.

9. A process for the production of polyglycidyl urazole compounds as claimed in Claim 1 and 3 to 8, characterized in that Z represents unsubstituted or monohydroxylated alkyl containing from 1 to 6 C-atoms or disubstituted alkyl containing from 3 to 7 C-atoms form the group comprising dihydroxyalkyl, halogenhydroxyalkyl, N-substituted aminohydroxyalkyl, alkoxyhydroxyalkyl, alkylmercaptohydroxyalkyl, alkylsulfoxyhydroxyalkyl and acyloxyhydroxyalkyl.

10. A process for the production of polyglycidyl urazole compounds as claimed in Claims 1 and 3 to 9, characterized in that Z is a disubstituted $C_3$-alkyl radical which contains the hydroxy group in the 2- or 3-position and the other substituents in the other two positions mentioned on the $C_3$-alkyl radical.

11. A process for the production of N-substituted polyglycidyl urazole compounds as claimed in Claims 1 to 10, characterized in that the three glycidyl radicals corresponding to general formula (II) are introduced into urazole in N-substitution, the triglycidyl urazole compound is, if desired, subjected to a partial reaction with water, alcohols, compounds containing a primary and/or secondary amino group, mercaptans, hydrogen sulfide, carboxylic acids, hydrogen halide or hydrogen or hydrogen-yielding compounds and, if desired, the mercapto compounds formed are converted into corresponding sulfoxy compounds and in that the two glycidyl radicals corresponding to general formula (II) are introduced in N-substitution into a urazole compound mono-N-substituted by the radical Z.

12. A process as claimed in Claim 11, characterized in that, for introducing the glycidyl radicals corresponding to general formula (II), urazole or a urazole compound monosubstituted by the radical Z is reacted with epihalohydrins and the halogydrin groups are subsequently dehydrohalogenated or the urazole compound used is first reacted with allyl halides and the allyl groups subsequently epoxidized.

13. A process as claimed in Claims 11 and 12, characterized in that the reaction of the urazole compounds with epihalohydrins or allyl halide is carried out in the presence of phase transfer catalysts.

14. N-substituted polyglycidyl urazole compounds as claimed in Claims 1 to 10 for use as cytostatic agents.

**Revendications pour les Etats contractants BE, LU, NL, SE**

1. Composés ge polyglycidyl-urazol N-substitués formule générale I

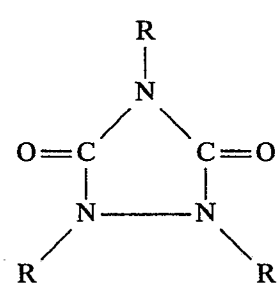

(I)

dans laquelle les radicaux R signifient un radical glycidyle de formule générale II:

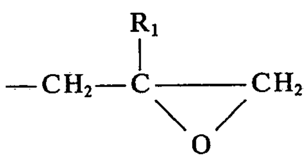

(II)

où $R_1$ signifie de l'hydrogène ou un radical alcoyle ayant 1 à 4 atomes de carbone, ou bien deux des radicaux R représentent un radical glycidyle de formule générale II tandis que le radical R restant est un radical Z qui contient 1 à 15 atomes de carbone, de l'hydrogène et éventuellement jusqu'à 3

hétéroatomes.

2. Composés de polyglycidyl-urazol de formule générale I, caractérisés en ce que les trois radicaux R signifient un radical glycidyle de formule générale II dans lequel $R_1$ est de l'hydrogène.

3. Composés de polyglycidyl-urazol selon la revendications 1, caractérisés en ce que deux des radicaux R signifient un radical glycidyle de formule II dans lequel $R_1$ est de préférence de l'hydrogène et Z contient comme hétéroatomes O, N, S et/ou P.

4. Composés de polyglycidyl-urazol selon les revendications 1 et 3, caractérisés en ce que Z signifie alcoyle, aryle, aralcoyle, alcaryle ou cycloalcoyle, radicaux qui éventuellement peuvent aussi être de nature hétérocyclique, insaturés, et/ou qui peuvent être substitués par au moins un des substituants suivants: halogène, hydroxyle, amino, amino N-substitué, mercapto, alcoylmercapto, arylmercapto, alcoylsulfoxy, arylsulfoxy, alcoxy, aryloxy, acyloxy et un radical hétérocyclique.

5. Composés de polyglycidyl-urazol selon les revendications 1, 3 et 4, caractérisés en ce que le radical Z présente au plus 12 atomes de carbone et en particulier au plus 8 atomes de carbone.

6. Composés de polyglycidyl-urazol selon les revendications 1, 3 à 5, caractérisés en ce que le radical Z est un radical alcoyle éventuellement substitué ayant jusqu'à 10 atomes de carbone, de préférence jusqu'à 6 atomes de carbone, en particulier jusqu'à 3 atomes de carbone.

7. Composés de polyglycidyl-urazol selon les revendications 1, 3 à 6, caractérisés en ce que le radical Z est un radical alcoyle substitué par au moins un groupe hydroxyle.

8. Composés de polyglycidyl-urazol selon les revendications 1, 3 à 7, caractérisés en ce que Z est un radical hydroxyalcoyle qui présente au moins un autre des substituants suivants: hydroxyle, halogène, amino, N-substitué, mercapto, alcoylmercapto, arylmercapto, alcoylsulfoxy, arylsulfoxy, alcoxy, aryloxy et acyle.

9. Composés de polyglycidyl-urazol selon les revendications 1, 3 à 8, caractérisés en ce que Z est un alcoyle non substitué ou monohydroxylé ayant 1 à 6 atomes de carbone ou un alcoyle disubstitué ayant 3 à 7 atomes de carbone appartenant au groupe dihydroxyalcoyle, halogénohydroxyalcoyle, aminohydroxyalcoyle N-substitué, alcoxyhydroxyalcoyle, alcoylmercaptohydroxyalcoyle, alcoylsulfoxyhydroxylcoyle et acyloxyhydroxyalcoyle.

10. Composés de polyglycidyl-urazol selon les revendications 1, 3 à 9, caractérisés en ce que Z est un radical alcoyle en $C_3$ disubstitué, qui présente le groupe hydroxy en position 2 ou 3 et les autres substituants dans les autres des deux positions citées sur le radical alcoyle en $C_3$.

11. Procédé de fabrication de composés de polyglycidyl-urazol N-substitués selon les revendications 1 à 10, caractérisé en ce qu'on introduit les trois radicaux glycidyle de formule générale II dans l'urazol en substitution N, on soumet éventuellement le composé de triglycidyl-urazol à une réaction partielle avec de l'eau, des alcools, des composés ayant un groupe amino primaire et/ou secondaire, des mercaptans, de l'hydrogène sulfuré, des acides carboxyliques, un hydracide halogéné ou de l'hydrogène ou des composés fournissant de l'hydrogène, en ce qu'on convertit éventuellement les mercaptocomposés formés en composés sulfoxy correspondants et en ce qu'on introduit dans un composé d'urazol mono-N-substitué par le radical Z les deux radicaux glycidyle de formule générale II en substitution N.

12. Procédé selon la revendication 11, caractérisé en ce que pour l'introduction des radicaux glycidyle de formule II, on fait réagir de l'urazol ou un composé d'urazol monosubstitué par le radical Z avec des épihalohydrines et l'on soumet ensuite les groupes halogydrine à une déshydrohalogénation, ou bien on fait d'abord réagir le composé d'urazol employé avec des halogénures d'allyle et l'on époxyde ensuite les groupes allyle.

13. Procédé selon les revendications 11 et 12, caractérisé en ce qu'on effectue la réaction des composés d'urazol avec les épihalohydrines ou avec un halogénure d'allyle en présence de catalyseurs de transfert de phase.

14. Composés de polyglycidyl-urazol N-substitués selon les revendications 1 à 10 en vue de l'application en tant qu'agents cytostatiques.

**Revendications pour les Etats contractants: CH, DE, FR, GB, IT, LI**

1. Composés de polyglycidyl-urazol N-substitués de formule générale I:

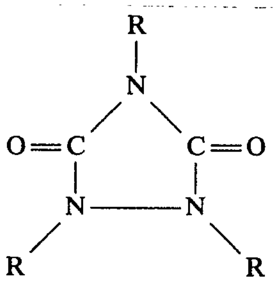

(I)

dans laquelle les radiaux R signifient un radical glycidyle de formule générale II:

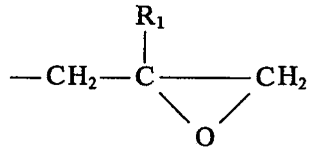

(II)

où $R_1$ est de l'hydrogène ou un radical alcoyle ayant 1 à 4 atomes de carbone, ou bien deux des radicaux R signifient un radical glycidyle de formule générale II et alors le radical R restant est un radical Z qui contient 1 à 15 atomes de carbone, de l'hydrogène et éventuellement jusqu'à 3 hétéroatomes, en vue de l'application en tant qu'agents cytostatiques.

2. Composés de polyglycidyl-urazol N-substitués selon la revendication 1, dans lequels les 3 radicaux R signifient un radical glycidyle de formule générale II, dans laquelle $R_1$ est de l'hydrogène, en vue de l'application en tant qu'agents cytostatiques.

3. Composés de polyglycidyl-urazol N-substitués de formule générale I

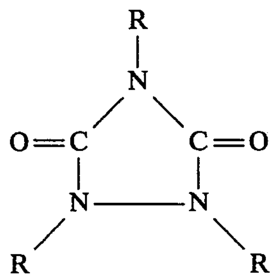

(I)

dans laquelle les radicaux R signifient un radical glycidyle de formule générale II

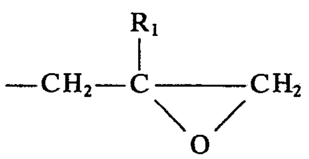

(II)

où $R_1$ est de l'hydrogène ou un radical alcoyle ayant 1 à 4 atomes de carbone, ou bien deux des radicaux R signifient un radical glycidyle de formule générale II et alors le radical R restant est un radical Z qui contient 1 à 15 atomes de carbone, de l'hydrogène et éventuellement jusqu'à 3 hétéroatomes, étant exceptés les composés qui contiennent 3 radicaux glycidyle dans lesquels $R_1$ est de l'hydrogène ou le groupe méthyle.

4. Composés de polyglycidyl-urazol selon la revendication 3, caractérisés en ce que 2 des radicaux R signifient un radical glycidyle de formule II, où $R_1$ est de préférence de l'hydrogène et Z contient O, N, S et/ou P comme hétéroatomes.

5. Composés de polyglycidyl-urazol selon la revendication 3, caractérisés en ce que Z signifie alcoyle, aryle, aralcoyle, alcaryle ou cycloalcoyle, radicaux qui éventuellement peuvent être de nature hétérocyclique, insaturés et/ou qui peuvent être substitués par au moins un des substituants suivants: halogène, hydroxyle, amino, amino N-substitués, mercapto, alcoylmercapto, arylmercapto, alcoylsulfoxy, arylsulfoxy, alcoxy, aryloxy, acyloxy et un radical hétérocyclique.

6. Composés de polyglycidyl-urazol selon les revendications 3 et 4, caractérisés en ce que le radical Z ne présente pas plus de 12 atomes de carbone et particulièrement pas plus de 8 atomes de carbone.

7. Composés de polyglycidyl-urazol selon les revendications 3 à 5, caractérisés en ce que le radical Z est un radical alcoyle éventuellement substitué ayant jusqu'à 10 atomes de carbone, de préférence jusqu'à 6 atomes de carbone, en particulier jusqu'à 3 atomes de carbone.

8. Composés de polyglycidyl-urazol selon les revendications 3 à 6, caractérisés en ce que le radical Z est un radical alcoyle substitué par au moins un groupe hydroxyle.

9. Composés de polyglycidyl-urazol selon les revendications 3 à 7, caractérisés en ce que Z est un radical hydroxyalcoyle présente au moins un autre des substituants suivants: hydroxyle, halogène, amino, amino N-substitué, mercapto, alcoylmercapto, arylmercapto, alcoylsulfoxy, arylsulfoxy, alcoxy, aryloxy et acyle.

10. Composés de polyglycidyl-urazol selon les revendications 3 à 8, caractérisés en ce que Z est un alcoyle non substitué ou monohydroxylé ayant 1 à 6 atomes de carbone, ou un alcoyle disubstitué ayant 3 à 7 atomes de carbone appartenant au groupe dihydroxyalcoyle, halogénohydroxyalcoyle, aminohydroxyalcoyle N-substitué, alcoxyhydroxyalcoyle, alcoylmercaptohydroxyalcoyle, alcoylsulfoxyhydroxyalcoyle et acyloxyhydroxyalcoyle.

11. Composés de polyglycidyl-urazol selon les revendications 3 à 9, caractérisés en ce que Z est

radical alcoyle en $C_3$ disubstitué, qui présente le groupe hydroxy en position 2 ou 3 et les autres substituants dans les autres des deux positions citées sur le radical alcoyle en $C_3$.

12. Procédé de fabrication de composés de polyglycidyl-urazol N-substitués selon les revendications 3 à 11, caractérisé en ce qu'on soumet du triglycidylurazol à une réaction partielle avec de l'eau, des alcools, des composés ayant un groupe amino primaire et/ou secondaire, des mercaptans, de l'hydrogène sulfuré, des acides carboxyliques, un hydracide halogéné ou de l'hydrogène ou des composés fournissant de l'hydrogène, on convertit éventuellement les mercaptocomposés formés en composés sulfoxy correspondants ou on introduit dans un composé urazol mono-N-substitué par le radical Z les deux radicaux glycidyle de formule générale II en substitution N.

13. Procédé selon la revendication 12, caractérisé en ce que pour l'introduction des radicaux glycidyle de formule générale II on fait d'abord réagir le composé d'urazol mis en jeu avec des halogénures d'allyle et l'on époxyde ensuite les groupes allyle.

14. Procédé selon les revendications 12 et 13, caractérisé en ce qu'on effectue la réaction des composés d'urazol avec des épihalohydrines ou un halogénure d'allyle en présence de catalyseurs de transfert de phase.

**Revendications pour l'etat contractant: AT**

1. Procédé de fabrication de composés de polyglycidyl-urazol N-substitués de formule générale I:

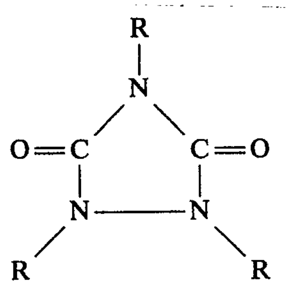

(I)

dans laquelle les radicaux R signifient un radical glycidyle de formule générale II:

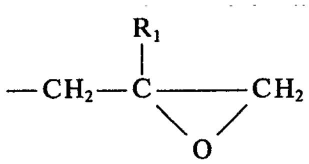

(II)

dans laquelle $R_1$ est de l'hydrogène ou un radical alcoyle ayant 1 à 4 atomes de carbone, ou bien deux des radicaux R signifient un radical glycidyle de formule générale II et alors le radical R restant est un radical Z, lequel contient 1 à 15 atomes de carbone, de l'hydrogène et éventuellement jusqu'à 3 hétéroatomes.

2. Procédé de fabrication de composés de polyglycidyl-urazol de formule générale I, caractérisé en ce que les trois radicaux R signifient un radical glycidyle de formule générale II, dans laquelle $R_1$ est de l'hydrogène.

3. Procédé de fabrication de composés de polyglycidyl-urazol selon la revendication 1, caractérisé en ce que deux des radicaux R signifient un radical glycidyle de formule II, dans laquelle $R_1$ est de préférence de l'hydrogène et Z contient O, N, S et/ou P comme hétéroatomes.

4. Procédé de fabrication de composés de polyglycidyl-urazol selon les revendications 1 et 3, caractérisé en ce que Z signifie un alcoyle, aryle, aralcoyle, alcaryle ou cycloalcoyle, radicaux qui le cas échéant peuvent être aussi de nature hétérocyclique, insaturés et/ou substitués par au moins un des substituants suivants: halogène, hydroxyle, amino N-substitué, mecapto, alcoylmercapto, arylmercapto, alcoylsulfoxy, arylsulfoxy, alcoxy, arylcoxy, acyloxy et un radical hétérocyclique.

5. Procédé de fabrication de composés de polyglycidyl-urazol selon les revendications 1, 3 et 4, caractérisé en ce que le radical Z ne présente pas plus de 12 atomes de carbone et en particulier pas plus de 8 atomes de carbone.

6. Procédé de fabrication de composés de polyglycidyl-urazol selon les revendications 1, 3 à 5, caractérisé en ce que le radical Z est un radical alcoyle éventuellement substitué ayant jusqu'à 10 atomes de carbone, de préférence jusqu'à 6 atomes de carbone, en particulier jusqu'à 3 atomes de carbone.

7. Procédé de fabrication de composés de polyglycidyl-urazol selon les revendications 1, 3 à 6, caractérisé en ce que le radical Z est un radical alcoyle substitué par au moins un groupe hydroxyle.

8. Procédé de fabrication de composés de polyglycidyl-urazol selon les revendications 1, 3 à 7, caractérisé en ce que Z est un radical hydroxyalcoyle qui présente au moins un autre des substituants

suivants: hydroxyle, halogène, amino, amino-N-substitué, mercapto, alcoylmercapto, arylmercapto, alcoylsulfoxy, arylsulfoxy, alcoxy, aryloxy et acyle.

9. Procédé de fabrication de composés de polyglycidyl-urazol selon les revendications 1, 3 à 8, caractérisé en ce que Z est un alcoyle non substitué ou monohydroxylé ayant 1 à 6 atomes de carbone, ou un alcoyle disubstitué ayant 3 à 7 atomes de carbone appartenant au groupe dihydroxyalcoyle, halogénohydroxyalcoyle, aminohydroxyalcoyle N-substitué, alcoxyhydroxyalcoyle, alcoyl mercapto-hydroxyalcoyle, alcoylsulfoxyhydroxyalcoyle et acyloxyhydroxyalcoyle.

10. Procédé de fabrication de composés de polyglycidyl-urazol selon les revendications 1, 3 à 9, caractérisé en ce que Z est un radical alcoyle en $C_3$ disubstitué qui présente le group hydroxy en position 2 ou 3 et les autres substituants dans les autres des deux positions citées sur le radical alcoyle en $C_3$.

11. Procédé de fabrication de composés de polyglycidyl-urazol selon les revendications 1 à 10, caractérisé en ce qu'on introduit les trois radicaux glycidyle de formule générale II dans l'urazol en N-substitution, en ce qu'on soumet le cas échéant le composé de triglycidyl-urazol à une réaction partielle avec de l'eau, des alcools, des composés ayant un groupe amino primaire et/ou secondaire, des mercaptans, de l'hydrogène sulfuré, des acides carboxyliques, un hydracide halogéné ou de l'hydrogne ou composés fournissant de l'hydrogène, en ce qu'on convertit au besoin les mercaptocomposés formés en sulfoxy composés correspondants et en ce qu'on introduit dans un composé d'urazol mono-N-substitué par le radical Z les deux radicaux glycidyle de formule générale II en substitution N.

12. Procédé selon la revendication 11, caractérisé en ce que pour l'introduction des radicaux glycidyle de formule générale II, on fait réagir l'urazol ou un composé d'urazol monosubstitué par le radial Z avec des épihalohydrines et en le soumettant par la suite à une déshydrohalogénation des groupes halohydrine, ou bien en ce qu'on fait d'abord réagir le composé d'urazol mis en jeu avec des halogénures d'allyle et l'on époxyde ensuite les groupes allyle.

13. Procédé selon les revendications 11 et 12, caractérisé en ce qu'on effectue la réaction des composés d'urazol avec des épihalohydrines ou un halogénure d'allyle en présence de catalyseurs de transfert de phase.

14. Composés de polyglycidyl-urazol N-substitués selon les revendications 1 à 10 en vue de l'application comme agents cytostatiques.